(19) Europäisches Patentamt
European Patent Office
Office européen des brevets

(11) EP 1 493 796 A1

(12) EUROPEAN PATENT APPLICATION
published in accordance with Art. 158(3) EPC

(43) Date of publication:
05.01.2005 Bulletin 2005/01

(21) Application number: 03712954.1

(22) Date of filing: 26.03.2003

(51) Int Cl.7: C09K 11/06, H05B 33/14, H05B 33/22

(86) International application number:
PCT/JP2003/003670

(87) International publication number:
WO 2003/080762 (02.10.2003 Gazette 2003/40)

(84) Designated Contracting States:
AT BE BG CH CY CZ DE DK EE ES FI FR GB GR HU IE IT LI LU MC NL PT RO SE SI SK TR

(30) Priority: 26.03.2002 JP 2002085642

(71) Applicants:
- Japan Science and Technology Agency
Kawaguchi-shi, Saitama 332-0012 (JP)
- Asahi Denka Co., Ltd.
Tokyo 116-8553 (JP)

(72) Inventors:
- TAKAHASHI, Tamotsu
Sapporo-shi, Hokkaido 064-0913 (JP)
- MUSHA, Kiyoshi, c/o ASAHI DENKA CO., LTD.
Tokyo 116-8553 (JP)
- SAKAMAKI, Koichi, c/o ASAHI DENKA CO., LTD.
Tokyo 116-8553 (JP)
- SHOJI, Yoshikazu, c/o ASAHI DENKA CO., LTD.
Tokyo 116-8553 (JP)

(74) Representative: Helbing, Jörg, Dr. Dipl.-Chem.
Patentanwälte
von Kreisler-Selting-Werner,
Postfach 10 22 41
50462 Köln (DE)

(54) FUNCTIONAL THIN FILM

(57) The present invention provides organic electroluminescent elements selected from polyacene derivatives represented by general formula (I) below:

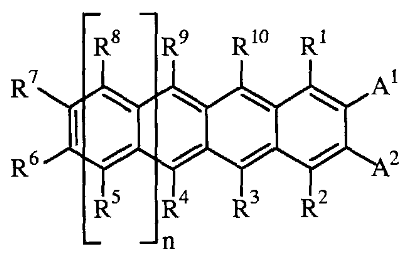

(I)

[wherein, $R^1$, $R^2$, $R^3$, $R^4$, $R^5$, $R^6$, $R^7$, $R^8$, $R^9$, $R^{10}$, $A^1$ and $A^2$, which are the same or different, each represents hydrogen atom, a hydrocarbon group, etc., and n denotes an integer of not less than 1]. The organic electroluminescent elements of the present invention can provide materials for organic electroluminescent elements and organic electroluminescent elements, which are excellent in stability, durability, luminance and luminance efficiency.

EP 1 493 796 A1

## Description

TECHNICAL FIELD

The present invention relates to materials for organic electroluminescent elements and organic electroluminescent elements.

BACKGROUND ART

Heretofore, inorganic electroluminescent elements have been used as panel light sources such as back light, etc. However, high voltage by alternating current is required to drive these electroluminescent elements. Recently, organic electroluminescent elements (organic EL elements) using organic compounds as luminescent materials were developed [Appl. Phys. Lett., 51, 913 (1987)]. An organic electroluminescent element is an element having the structure that a thin film containing a fluorescent organic compound is sandwiched between an anode and a cathode, in which electrons and holes are injected into the thin film for recombination to generate excitons, and the light released when the excitons are inactivated is utilized to emit light. The organic electroluminescent element enables to emit light with a low voltage of direct current of several to several tens and by selecting the kind of fluorescent organic compounds, are capable of emitting various colors (e.g., red, blue and green colors). The organic electroluminescent element having such features are expected to be applied for various luminescent elements, display devices, etc. In general, however, organic electroluminescent elements encounter disadvantages such as poor stability or durability, etc. In addition, these elements are poor in luminance and insufficient for practical applications.

It was proposed to use as a hole injection transport material 4,4'-bis[N-phenyl-N-(3"-methylphenyl)amino] biphenyl [Jpn. J. Appl. Phys., 27, L269 (1988)]. However, this organic electroluminescent element also involves disadvantages such as poor stability, poor durability, etc. To enhance the luminance, organic electroluminescent elements that as a luminescent layer, for example, tris(8-quinolinolato)aluminum was used as a host compound and a coumarin derivative or a pyran derivative was used as a guest compound (dopant) were proposed [J.Appl. Phys., 65, 3610 (1989)]. Also, organic electroluminescent elements that in the luminescent layer bis(2-methyl-8-quinolinolato)(4-phenylphenolato)aluminum was used as a host compound and an acridone derivative (e.g., N-methyl-2-methoxyacridone) was used as a guest compound were proposed (Japanese Laid-Open Publication No. H08-67873). However, it is hard to say that these electroluminescent elements have a sufficient luminance. Now, a more improved organic electroluminescent element has been desired.

Furthermore, materials for the electron injection transport are not satisfactory, either, and a more improved organic electroluminescent element has been desired.

DISCLOSURE OF THE INVENTION

An object of the present invention is to provide a material for an organic electroluminescent element and the organic electroluminescent element, which is excellent in stability, durability, luminance and luminance efficiency.

The present inventors have made extensive investigations on materials for organic electroluminescent elements and as a result, have come to accomplish the present invention.

That is, the present invention provides a material for the organic electroluminescent element selected from a polyacene derivative represented by general formula (I) below:

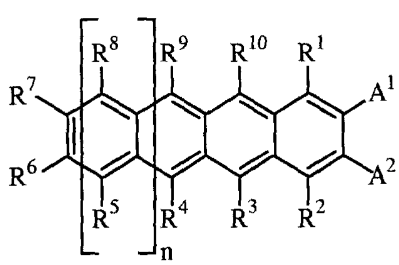

(I)

[wherein $R^1$, $R^2$, $R^3$, $R^4$, $R^5$, $R^6$, $R^7$, $R^8$, $R^9$ and $R^{10}$, which may be the same or different, independently represents hydrogen atom; a $C_1$-$C_{40}$ hydrocarbon group which may optionally be substituted; a $C_1$-$C_{40}$ alkoxy group which may optionally be substituted, a $C_6$-$C_{40}$ aryloxy group which may optionally be substituted; an amino group which may optionally be substituted, hydroxy group, or a silyl group which may optionally be substituted; provided that $R^6$ and $R^7$

may be cross-bridged with one another to form a $C_4$-$C_{40}$ saturated or unsaturated ring, and the saturated or unsaturated ring may be intervened by oxygen atom, sulfur atom or a group shown by formula: -N($R^{11}$)- (wherein $R^{11}$ is hydrogen atom or a hydrocarbon group) and may optionally be substituted;

$A^1$ and $A^2$, which may be the same or different, independently represents hydrogen atom; a halogen atom; a $C_1$-$C_{40}$ hydrocarbon group which may optionally be substituted; a $C_1$-$C_{40}$ alkoxy group which may optionally be substituted; a $C_6$-$C_{40}$ aryloxy group which may optionally be substituted; a $C_7$-$C_{40}$ alkylaryloxy group which may optionally be substituted; a $C_2$-$C_{40}$ alkoxycarbonyl group which may optionally be substituted; a $C_7$-$C_{40}$ aryloxycarbonyl group which may optionally be substituted; cyano group (-CN); carbamoyl group (-C(=O)$NH_2$); a haloformyl group (-C(=O) -X, wherein X represents a halogen atom); formyl group (-C(=O)-H); isocyano group; isocyanate group; thiocyanate group or thioisocyanate group; provided that $A^1$ and $A^2$ may be cross-bridged with each other to form a ring shown by formula: -C(=O)-B-C(=O)- (wherein B is oxygen atom or a group shown by formula -N($B^1$)-(wherein $B^1$ is hydrogen atom, a $C_1$-$C_{40}$ hydrocarbon group or a halogen atom));

n is an integer of at least 1;

provided that, the case wherein $R^1$, $R^2$, $R^3$, $R^4$, $R^5$, $R^6$, $R^7$, $R^8$, $R^9$, $R^{10}$, $A^1$ and $A^2$ are all hydrogen atoms is excluded,

the case wherein $R^7$ and $A^1$ are methoxy groups or $A^2$ and $R^6$ are methoxy groups is excluded,

when n is 1;

at least $R^1$, $R^2$, $R^4$ and $R^9$ are groups other than hydrogen atom, or at least $R^3$, $R^5$, $R^8$ and $R^{10}$ are groups other than hydrogen atom,

the case wherein $R^3$ and $R^{10}$ or $R^4$ and $R^9$ are an aryl group which may optionally be substituted is excluded; and,

when n is 2, the formula (I) described above is a pentacene derivative represented by formula (Ia) below:

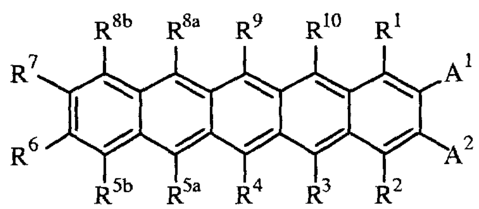

(Ia)

(wherein $A^1$ and $A^2$ have the same significance as described above; $R^1$, $R^2$, $R^3$, $R^4$, $R^{5a}$, $R^{5b}$, $R^6$, $R^7$, $R^{8a}$, $R^{8b}$, $R^9$ and $R^{10}$, which may be the same or different, independently represents hydrogen atom; a $C_1$-$C_{40}$ hydrocarbon group which may optionally be substituted; a $C_1$-$C_{40}$ alkoxy group which may optionally be substituted; a $C_6$-$C_{40}$ aryloxy group which may or unsaturatea ring may be intervened by oxygen atom, sulfur atom or a group represented by formula: -N ($R^{11}$)- (wherein $R^{11}$ is hydrogen atom or a hydrocarbon group) and may optionally be substituted), and the case in which at least one of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ is a diarylamine group is excluded].

The present invention further provides the material for organic electroluminescent elements described above, which is represented by general formula (I) wherein n is 1 or 2;

when n is 1, the following cases are excluded:

the case wherein $R^1$, $R^2$, $R^3$, $R^4$, $R^5$, $R^6$, $R^7$, $R^8$, $R^9$, $R^{10}$, $A^1$ and $A^2$ are all methyl groups;
the case wherein $R^3$, $R^4$, $R^9$ and $R^{10}$ are all aryl groups and $R^1$, $R^2$, $R^5$, $R^6$, $R^7$, $R^8$, $A^1$ and $A^2$ are all hydrogen atoms;
the case wherein $R^1$, $R^2$, $R^4$ and $R^9$ are all alkoxy groups or aryloxy groups and $R^3$, $R^5$, $R^6$, $R^7$, $R^8$, $R^{10}$, $A^1$ and $A^2$ are all hydrogen atoms; and,
the case wherein $R^3$, $R^5$, $R^8$ and $R^{10}$ are all alkoxy groups or aryloxy groups and $R^1$, $R^2$, $R^4$, $R^6$, $R^7$, $R^9$, $A^1$ and $A^2$ are all hydrogen atoms;

when n is 2, the following cases are excluded:

the case of the pentacene derivative represented by the formula (Ia) above wherein, in the formula (Ia), $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are all methyl groups; or $R^1$, $R^2$, $R^3$, $R^4$, $R^{5a}$, $R^{5b}$, $R^{8a}$, $R^{8b}$, $R^9$ and $R^{10}$ are all hydrogen atoms and at least one of $R^6$, $R^7$, $A^1$ and $A^2$ is an aryl group; or when at most 6 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom, any one of the groups other than hydrogen atom is methoxy group;
the case of the pentacene derivative represented by formula (Ib) below:

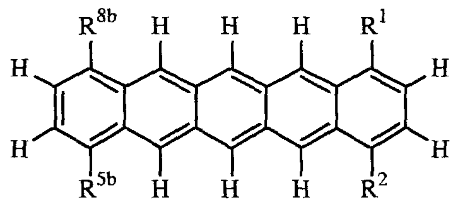

(Ib)

[wherein $R^1$, $R^2$, $R^{5b}$ and $R^{8b}$ are all alkoxy groups or aryloxy groups];

the case of the pentacene derivative represented by formula (Ic):

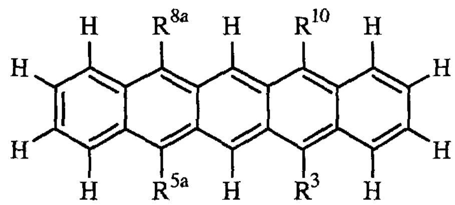

(Ic)

[wherein at least 2 of $R^3$, $R^{5a}$, $R^{8a}$ and $R^{10}$ are aryl groups or arylalkynyl groups, or at least one of $R^3$, $R^{5a}$, $R^{8a}$ and $R^{10}$ is an arylalkenyl group, or $R^3$, $R^{5a}$, $R^{8a}$ and $R^{10}$ are all alkoxy groups or aryloxy groups]; and,

the case of the pentacene derivative represented by formula (Id) below:

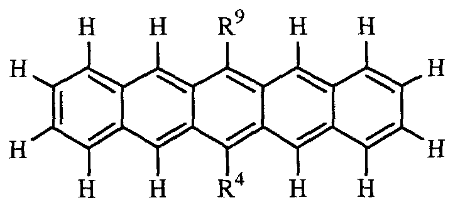

(Id)

[wherein $R^4$ and $R^9$ are hydrogen atom, a hydrocarbon group, an alkoxy group, an aryloxy group, or a halogen atom or hydroxy group].

The present invention further provides the materials for organic electroluminescent elements selected from the polyacene derivatives that in the formula (I), at least 5 of $R^1$, $R^2$, $R^3$, $R^4$, $R^5$, $R^6$, $R^7$, $R^8$, $R^9$, $R^{10}$, $A^1$ and $A^2$ are groups other than hydrogen atom.

The present invention still further provides the materials for organic electroluminescent elements selected from the polyacene derivatives that in the formula (I), at least 6 of $R^1$, $R^2$, $R^3$, $R^4$, $R^5$, $R^6$, $R^7$, $R^8$, $R^9$, $R^{10}$, $A^1$ and $A^2$ are groups other than hydrogen atom.

The present invention still further provides the material for organic electroluminescent elements selected from the polyacene derivatives that the polyacene derivatives are pentacene derivatives represented by the formula (Ia) above wherein at least 5 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom.

The present invention still further provides the materials for organic electroluminescent elements selected from the polyacene derivatives that in the formula (Ia), at least 6 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom.

The present invention still further provides the materials for organic electroluminescent elements selected from the polyacene derivatives that in the formula (Ia), at least 7 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom.

The present invention still further provides the materials for organic electroluminescent elements selected from the polyacene derivatives that in the formula (Ia), at least 8 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom.

The present invention still further provides the materials for organic electroluminescent elements selected from the polyacene derivatives that in the formula (Ia), at least 9 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom.

The present invention still further provides the materials for organic electroluminescent elements selected

from the polyacene derivatives that in the formula (Ia), at least 10 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom.

The present invention still further provides the materials for organic electroluminescent elements selected from the polyacene derivatives that in the formula (I), any combination of $R^1$ and $R^2$, $R^3$ and $R^{10}$, $R^4$ and $R^9$, $R^5$ and $R^8$, $R^6$ and $R^7$ as well as $A^1$ and $A^2$ is the same substituent.

The present invention still further provides the materials for organic electroluminescent elements selected from the polyacene derivatives that in the formula (Ia), any combination of $R^1$ and $R^2$, $R^3$ and $R^{10}$, $R^4$ and $R^9$, $R^{5a}$ and $R^{8a}$, $R^{5b}$ and $R^{8b}$, $R^6$ and $R^7$ as well as $A^1$ and $A^2$ is the same substituent.

The present invention still further provides the materials for organic electroluminescent elements selected from the polyacene derivatives that in the formula (I), any one of $R^1$, $R^2$, $R^3$, $R^4$, $R^5$, $R^6$, $R^7$, $R^8$, $R^9$ and $R^{10}$ is a $C_1$-$C_{40}$ hydrocarbon group which may optionally be substituted; a $C_1$-$C_{40}$ alkoxy group which may optionally be substituted, or a $C_6$-$C_{40}$ aryloxy group which may optionally be substituted.

The present invention still further provides the materials for organic electroluminescent elements selected from the polyacene derivatives that in the formula (Ia), any one of $R^1$, $R^2$, $R^3$, $R^4$, $R^{5a}$, $R^{5b}$, $R^6$, $R^7$, $R^{8a}$, $R^{8b}$, $R^9$ and $R^{10}$ is a $C_1$-$C_{40}$ hydrocarbon group which may optionally be substituted; a $C_1$-$C_{40}$ alkoxy group which may optionally be substituted, or a $C_6$-$C_{40}$ aryloxy group which may optionally be substituted.

The present invention still further provides the materials for organic electroluminescent elements selected from the polyacene derivatives that in the formula (I), $R^1$, $R^2$, $R^4$ and $R^9$, which may be the same or different, independently represents a $C_1$-$C_{40}$ alkyl group which may optionally be substituted or a $C_6$-$C_{18}$ aryl group which may optionally be substituted; $A^1$ and $A^2$, which may be the same or different, independently represents a $C_2$-$C_{40}$ alkoxycarbonyl group which may optionally be substituted; and n is 1.

The present invention still further provides the materials for organic electroluminescent elements selected from the polyacene derivatives that in the formula (I), $A^1$, $A^2$, $R^1$, $R^2$, $R^4$ and $R^9$, which may be the same or different, independently represents a $C_1$-$C_{40}$ alkyl group which may optionally be substituted or a $C_6$-$C_{18}$ aryl group which may optionally be substituted; and n is 1.

The present invention still further provides the materials for organic electroluminescent elements selected from the polyacene derivatives that in the formula (I), $R^3$, $R^5$, $R^6$, $R^7$, $R^8$ and $R^{10}$, which may be the same or different, independently represents a $C_1$-$C_{40}$ alkyl group which may optionally be substituted or a $C_6$-$C_{18}$ aryl group which may optionally be substituted; $A^1$ and $A^2$, which may be the same or different, independently represents a halogen atom; and n is 1.

The present invention still further provides the materials for organic electroluminescent elements selected from the polyacene derivatives that, when the polyacene derivatives are the pentacene derivatives represented by the formula (Ia) above, $A^1$ and $A^2$ represent a $C_2$-$C_{40}$ alkoxycarbonyl group which may optionally be substituted, and $R^1$, $R^2$, $R^4$, $R^{5b}$, $R^6$, $R^7$, $R^{8b}$, $R^9$ represent a $C_1$-$C_{40}$ alkyl group which may optionally be substituted, or a $C_6$-$C_{18}$ aryl group which may optionally be substituted.

The present invention still further provides the materials for organic electroluminescent elements selected from the polyacene derivatives that, when the polyacene derivatives are the pentacene derivatives represented by the formula (Ia) above, $A^1$, $A^2$, $R^1$, $R^2$, $R^4$, $R^{5b}$, $R^6$, $R^7$, $R^{8b}$, $R^9$ represent a $C_1$-$C_{40}$ alkyl group which may optionally be substituted or a $C_6$-$C_{18}$ aryl group which may optionally be substituted.

The present invention still further provides the materials for organic electroluminescent elements selected from the polyacene derivatives, when the polyacene derivatives are the pentacene derivatives represented by the formula (Ia) above, $A^1$ and $A^2$ represent a halogen atom and, $R^3$, $R^{5a}$, $R^{8a}$ and $R^{10}$ represent a $C_1$-$C_{40}$ alkyl group which may optionally be substituted or a $C_6$-$C_{18}$ aryl group which may optionally be substituted.

The present invention still further provides the organic electroluminescent elements comprising a pair of electrodes and at least one layer containing at least one of the polyacene derivatives above, which is sandwiched between the electrodes.

The present invention still further provides the organic electroluminescent element s, wherein the layer containing at least one of the polyacene derivatives is a luminescent layer.

The present invention still further provides the organic electroluminescent element s, wherein the layer containing at least one of the polyacene derivatives is a hole injection transport layer.

The present invention still further provides the organic electroluminescent elements, wherein the layer containing at least one of the polyacene derivatives is an electron injection transport layer.

The present invention still further provides the organic electroluminescent elements, which further contains at least one of a polycyclic aromatic compound, a luminescent organic metal-complex or a triarylamine derivative.

The present invention still further provides the organic electroluminescent elements, wherein 0.1 to 40% by weight of the polyacene derivatives described above is contained in at least one layer.

BRIEF DESCRIPTION OF DRAWINGS

FIG. 1 is a schematic view of the structure of an embodiment (A) of the organic electroluminescent element.
FIG. 2 is a schematic view of the structure of an embodiment (B) of the organic electroluminescent element.
FIG. 3 is a schematic view of the structure of an embodiment (C) of the organic electroluminescent element.
FIG. 4 is a schematic view of the structure of an embodiment (D) of the organic electroluminescent element.
FIG. 5 is a schematic view of the structure of an embodiment (E) of the organic electroluminescent element.
FIG. 6 is a schematic view of the structure of an embodiment (F) of the organic electroluminescent element.
FIG. 7 is a schematic view of the structure of an embodiment (G) of the organic electroluminescent element.
FIG. 8 is a schematic view of the structure of an embodiment (H) of the organic electroluminescent element.

BEST MODE FOR CARRYING OUT THE INVENTION

Hereinafter the present invention will be described in detail.

The material for organic electroluminescent element of the present invention is the polyacene derivative represented by general formula (I). The organic electroluminescent element of the present invention comprises a pair of electrodes and at least one of the polyacene derivatives represented by general formula (I), sandwiched between the electrodes. The polyacene derivative represented by general formula (I), which is the material for the organic electroluminescent element of the present invention (hereinafter also simply referred to as the polyacene derivative), has the polyacene structure represented by general formula (I):

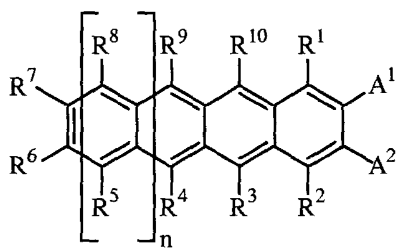

(I)

[wherein $R^1$, $R^2$, $R^3$, $R^4$, $R^5$, $R^6$, $R^7$, $R^8$, $R^9$, $R^{10}$, $A^1$ and $A^2$ have the same significance as described above].

In the formula (I) above, $R^1$, $R^2$, $R^3$, $R^4$, $R^5$, $R^6$, $R^7$, $R^8$, $R^9$ and $R^{10}$, which may be the same or different, independently represents hydrogen atom; a $C_1$-$C_{40}$ hydrocarbon group which may optionally be substituted; a $C_1$-$C_{40}$ alkoxy group which may optionally be substituted, a $C_6$-$C_{40}$ aryloxy group which may optionally be substituted; amino group, hydroxy group or a silyl group.

In the specification, the $C_1$-$C_{40}$ hydrocarbon group may be a saturated or unsaturated non-cyclic group, or may be a saturated or unsaturated cyclic group. When the $C_1$-$C_{40}$ hydrocarbon group is a non-cyclic group, it may be linear or branched. The $C_1$-$C_{40}$ hydrocarbon group includes a $C_1$-$C_{40}$ alkyl group, a $C_2$-$C_{40}$ alkenyl group, a $C_2$-$C_{40}$ alkynyl group, a $C_3$-$C_{40}$ allyl group, a $C_4$-$C_{40}$ alkyldienyl group, a $C_4$-$C_{40}$ polyenyl group, a $C_6$-$C_{18}$ aryl group, a $C_6$-$C_{40}$ alkylaryl group, a $C_6$-$C_{40}$ arylalkyl group, a $C_4$-$C_{40}$ cycloalkyl group, a $C_4$-$C_{40}$ cycloalkenyl group, and the like.

The $C_1$-$C_{40}$ alkyl group, $C_2$-$C_{40}$ alkenyl group, $C_2$-$C_{40}$ alkynyl group, $C_3$-$C_{40}$ allyl group, $C_4$-$C_{40}$ alkyldienyl group and $C_4$-$C_{40}$ polyenyl group are preferably a $C_1$-$C_{20}$ alkyl group, a $C_2$-$C_{20}$ alkenyl group, a $C_2$-$C_{20}$ alkynyl group, a $C_3$-$C_{20}$ allyl group, a $C_4$-$C_{20}$ alkyldienyl group and a $C_4$-$C_{20}$ polyenyl group, respectively, and more preferably, a $C_1$-$C_{10}$ alkyl group, a $C_2$-$C_{10}$ alkenyl group, a $C_2$-$C_{10}$ alkynyl group, a $C_3$-$C_{10}$ allyl group, a $C_4$-$C_{10}$ alkyldienyl group and a $C_4$-$C_{10}$ polyenyl group, respectively.

Examples of the alkyl group useful for practicing the present invention, which may optionally be substituted include, but not limited to, methyl, ethyl, propyl, n-butyl, t-butyl, dodecanyl, trifluoromethyl, perfluoro-n-butyl, 2,2,2-trifluoroethyl, benzyl, 2-phenoxyethyl, and the like.

Examples of the aryl group useful for practicing the present invention, which may optionally be substituted include, but not limited to, phenyl, 2-tolyl, 3-tolyl, 4-tolyl, naphthyl, biphenyl, 4-phenoxyphenyl, 4-fluorophenyl, 3-carbomethoxyphenyl, 4-carbomethoxyphenyl, etc.

Examples of the alkoxy group useful for practicing the present invention, which may optionally be substituted include, but not limited to, methoxy, ethoxy, 2-methoxyethoxy, t-butoxy, etc.

Examples of the aryloxy group useful for practicing the present invention, which may optionally be substituted include, but not limited to, phenoxy, naphthoxy, phenylphenoxy, 4-methylphenoxy, etc.

Examples of the amino group useful for practicing the present invention, which may optionally be substituted include, but not limited to, amino, dimethylamino, methylamino, methylphenylamino, phenylamino, etc.

The silyl group which may optionally be substituted include group s represented by formula: $-Si(R^{12})(R^{13})(R^{14})$ [wherein $R^{12}$, $R^{13}$ and $R^{14}$, which may be the same or different, independently represents a $C_1$-$C_{40}$ alkyl group which may optionally be substituted with a halogen atom; a $C_6$-$C_{40}$ arylalkyl group which may optionally be substituted with a halogen atom; a $C_1$-$C_{40}$ alkoxy group which may optionally be substituted with a halogen atom; or a $C_6$-$C_{40}$ arylalkyloxy group which may optionally be substituted with a halogen atom].

Examples of the silyl group useful for practicing the present invention, which may optionally be substituted include, but not limited to, trimethylsilyl, triethylsilyl, trimethoxysilyl, triethoxysilyl, diphenylmethylsilyl, triphenylsilyl, triphenoxysilyl, dimethylmethoxysilyl, dimethylphenoxysilyl, methylmethoxyphenyl, etc.

The $C_1$-$C_{40}$ hydrocarbon group, $C_1$-$C_{40}$ alkoxy group, $C_6$-$C_{40}$ aryloxy group, amino group, silyl group, etc. may optionally be substituted with a substituent and the substituent are a halogen atom, hydroxy group, amino group, etc.

Examples of the halogen atom include fluorine atom, chlorine atom, bromine atom and iodine atom. When the hydrogen atom(s) of the $C_1$-$C_{40}$ hydrocarbon group, $C_1$-$C_{40}$ alkoxy group, $C_6$-$C_{40}$ aryloxy group, etc. are substituted with fluorine atom(s), the solubility of the polyacene derivatives increases, which is preferred.

$R^6$ and $R^7$ may be cross-bridged with each other to form a $C_4$-$C_{40}$ saturated or unsaturated ring. The unsaturated ring may be an aromatic ring such as a benzene ring, etc. The ring formed by linking $R^6$ and $R^7$ together is preferably a 4-membered ring to a 16-membered ring, more preferably a 4-membered ring to a 12-membered ring. The ring may be an aromatic ring or an aliphatic ring. In this ring, a substituent such as a $C_1$-$C_{20}$ hydrocarbon group, a $C_1$-$C_{20}$ alkoxy group, a $C_6$-$C_{20}$ aryloxy group, amino group, hydroxy group or silyl group, etc. may be introduced.

The saturated or unsaturated ring may be intervened by oxygen atom, sulfur atom or a group shown by formula: $-N(R^{11})-$ [wherein $R^{11}$ is hydrogen atom or a hydrocarbon group]. Preferably, $R^{11}$ is hydrogen atom or a $C_1$-$C_6$ alkyl group, and more preferably, hydrogen atom or a $C_1$-$C_4$ alkyl group.

In the formula (I) described above, $A^1$ and $A^2$, which may be the same or different, independently represents hydrogen atom; a halogen atom; a $C_1$-$C_{40}$ hydrocarbon group which may optionally be substituted; a $C_1$-$C_{40}$ alkoxy group which may optionally be substituted, a $C_6$-$C_{40}$ aryloxy group which may optionally be substituted; a $C_7$-$C_{40}$ alkylaryloxy group which may optionally be substituted; a $C_2$-$C_{40}$ alkoxycarbonyl group which may optionally be substituted; a $C_7$-$C_{40}$ aryloxycarbonyl group which may optionally be substituted; cyano group (-CN); carbamoyl group ($-C(=O)NH_2$); a haloformyl group (-C(=O)-X, wherein X represents a halogen atom); formyl group (-C(=O)-H); isocyano group; isocyanate group, thiocyanate group or thioisocyanate group.

The cyano group (-CN); carbamoyl group ($-C(=O)NH_2$); a haloformyl group (-C(=O)-X, wherein X represents a halogen atom); formyl group (-C(=O)-H), isocyano group, isocyanate group, thiocyanate group or thioisocyanate group can be converted from, e.g., an alkoxycarbonyl group in a conventional manner of the organic chemistry. Also, carbamoyl group ($-C(=O)NH_2$), a haloformyl group (-C(=O)-X, wherein X represents a halogen atom), formyl group (-C(=O)-H), etc. can be converted into cyano group or the alkoxycarbonyl group, and vice versa.

$A^1$ and $A^2$ may be cross-bridged with one another to form a ring represented by formula: -C(=O)-B-C(=O)- [wherein B is oxygen atom or a group represented by formula: $-N(B^1)-$ (wherein $B^1$ is hydrogen atom, a $C_1$-$C_{40}$ hydrocarbon group or a halogen atom)].

For example, where $A^1$ and $A^2$ is the alkoxycarbonyl group, the groups can be converted into carboxy group in a conventional manner of the organic chemistry. And, the carboxyl group adjacent one another can be dehydrated to the carboxylic anhydride, namely, a ring represented by formula: -C(=O)-O-C(=O)-. Similarly, the carboxylic anhydride can be converted into the imide, i.e., a ring represented by formula: $-C(=O)-N(B^1)-C(=O)-$ ($B^1$ has the same significance as described above) in a conventional manner of the organic chemistry.

n is an integer of 1 or more. When n is 1 and 2, the derivative becomes a 4-membered ring and 5- membered ring, that is, a naphthacene derivative and a pentacene derivative, respectively, etc.

In the past, there was a tendency in condensed polycyclic aromatic compounds that the solubility decreased as the number of the aromatic ring increased. According to the process of producing these compounds later described, however, the solubility can be maintained by introducing various adequate substituents, even though the number of the aromatic ring in the condensed polycyclic aromatic compounds increases. Accordingly, n is not limited to 1 and 2 but may be an integer of 3 or more, an integer of 4 or more, or an integer of 5 or more. For example, the polyacene derivative in which 7 benzene rings are condensed (which corresponds to n = 4) is obtained.

n may be 200 or less, 100 or less, 80 or less, 50 or less, 30 or less, 20 or less, 15 or less, or 10 or less. For example, since the number of n increases by 2 at a time by applying the process described below, this scheme may be repeated. As described above, by adequately introducing substituents the solubility can be maintained so that the number of n can be increased.

In the present invention, the materials for organic electroluminescent elements selected from the polyacene derivatives of formula (I) described above wherein $R^1$, $R^2$, $R^3$, $R^4$, $R^5$, $R^6$, $R^7$, $R^8$, $R^9$ , $R^{10}$, $A^1$ and $A^2$ are all hydrogen

atoms are not included. The materials for organic electroluminescent elements selected from the polyacene derivatives of formula (I) described above wherein $R^7$ and $A^1$ are methoxy groups or $A^2$ and $R^6$ are methoxy groups are not included, either.

Further in the present invention, when n is 1 in the formula (I) above, at least $R^1$, $R^2$, $R^4$ and $R^9$ are groups other than hydrogen atom, or at least $R^3$, $R^5$, $R^8$ and $R^{10}$ are groups other than hydrogen atom. Furthermore, the materials for organic electroluminescent elements selected from the polyacene derivatives of the formula (I) above wherein, when n is 1, at least $R^3$ and $R^{10}$, or $R^4$ and $R^9$ are an aryl group which may optionally be substituted are not included in the present invention.

Furthermore, when n is 2 in the formula (I) described above and the formula (I) above is pentacene derivatives represented by the formula (Ia) below:

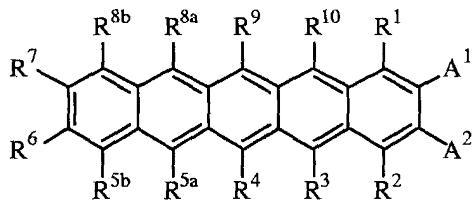

(Ia)

[wherein $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ have the same significance as described above, respectively].

The materials for organic electroluminescent elements selected from the pentacene derivatives wherein at least one of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ is a diarylamine group are not included.

In the present invention, preferably the polyacene derivatives represented by the formula (I) above wherein n is 1 are not intended to include the case wherein $R^1$, $R^2$, $R^3$, $R^4$, $R^5$, $R^6$, $R^7$, $R^8$, $R^9$, $R^{10}$, $A^1$ and $A^2$ are all methyl groups; the case wherein $R^3$, $R^4$, $R^9$ and $R^{10}$ are all aryl groups and $R^1$, $R^2$, $R^5$, $R^6$, $R^7$, $R^8$, $A^1$ and $A^2$ are all hydrogen atoms; the case wherein $R^1$, $R^2$, $R^4$ and $R^9$ are all alkoxy groups or aryloxy groups and $R^3$, $R^5$, $R^6$, $R^7$, $R^8$, $R^{10}$, $A^1$ and $A^2$ are all hydrogen atoms; and the case wherein $R^3$, $R^5$, $R^8$ and $R^{10}$ are all alkoxy groups or aryloxy groups and $R^1$, $R^2$, $R^4$, $R^6$, $R^7$, $R^9$, $A^1$ and $A^2$ are all hydrogen atoms.

In the present invention, preferably the polyacene derivatives represented by the formula (I) above wherein n is 2 are not intended to include the cases of (a'), (b'), (c') and (d').

(a') In the formula (Ia) described above, the case wherein $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are all methyl groups, or the case wherein $R^1$, $R^2$, $R^3$, $R^4$, $R^{5a}$, $R^{5b}$, $R^{8a}$, $R^{8b}$, $R^9$ and $R^{10}$ are all hydrogen atoms and at least one of $R^6$, $R^7$, $A^1$ and $A^2$ is an aryl group, or the case that when at most 6 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom, at least one of the aforesaid groups other than the hydrogen atom is methoxy group.

(b') The case of pentacene derivatives represented by formula (Ib) below:

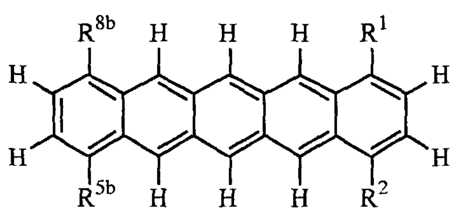

(Ib)

wherein $R^1$, $R^2$, $R^{5b}$ and $R^{8b}$ are all alkoxy groups or aryloxy groups.

(c') The case of pentacene derivatives represented by formula (Ic) below:

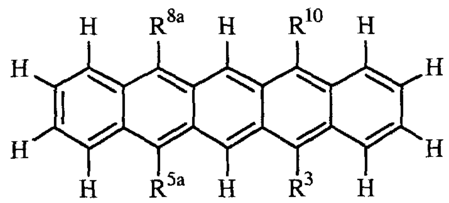

(Ic)

wherein at least 2 of $R^3$, $R^{5a}$, $R^{8a}$ and $R^{10}$ are aryl groups or arylalkynyl groups, or at least one of $R^3$, $R^{5a}$, $R^{8a}$ and $R^{10}$ is an arylalkenyl group, or $R^3$, $R^{5a}$, $R^{8a}$ and $R^{10}$ are all alkoxy groups or aryloxy groups.

(d') The case of pentacene derivatives represented by formula (Id) below:

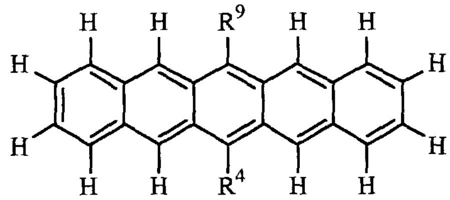

(Id)

wherein $R^4$ and $R^9$ represent hydrogen atom, a hydrocarbon group, an alkoxy group, an aryloxy group, or a halogen atom or hydroxy group.

In the present invention, the polyacene derivatives represented by the formula (I) described above are preferably those wherein at least 5 of $R^1$, $R^2$, $R^3$, $R^4$, $R^5$, $R^6$, $R^7$, $R^8$, $R^9$, $R^{10}$, $A^1$ and $A^2$ are groups other than hydrogen atom, more preferably at least 6 of them are groups other than hydrogen atom, and most preferably at least 8 of them are groups other than hydrogen atom. As will be later described, this is because, when dehydrogenation is carried out using the combination of a lithium dopant and a lithium-removing reagent, the yield occasionally decreases as the number of hydrogen atom in $R^1$, $R^2$, $R^3$, $R^4$, $R^5$, $R^6$, $R^7$, $R^8$, $R^9$, $R^{10}$, $A^1$ and $A^2$ increases.

When the polyacene derivatives represented by the formula (I) above are the pentacene derivatives shown by formula (Ia) below:

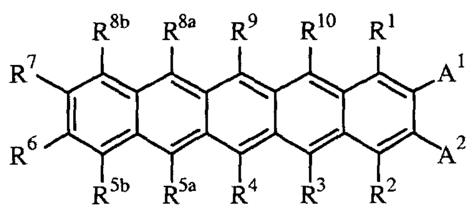

(Ia)

[wherein $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ have the same significance as described above], preferably at least 5 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom, more preferably at least 6 of them are groups other than hydrogen atom, preferably at least 7 of them are groups other than hydrogen atom, further more preferably at least 8 of them are groups other than hydrogen atom, much more preferably at least 9 of them are groups other than hydrogen atom, and most preferably at least 10 of them are groups other than hydrogen atom.

Further in the polyacene derivatives represented by the formula (I) above, the polyacene derivatives wherein the substituents are the same in any combination of $R^1$ and $R^2$, $R^3$ and $R^{10}$, $R^4$ and $R^9$, $R^5$ and $R^8$, $R^6$ and $R^7$ as well as $A^1$ and $A^2$ are preferred, and more preferably, the substituents are the same in all of the respective combinations. This is because synthesis of such polyacene derivatives becomes easy and the yield is improved.

For the same reason, in the polyacene derivatives represented by the formula (Ia) above, preferred are the polyacene derivatives wherein the substituents are the same in any combination of $R^1$ and $R^2$, $R^3$ and $R^{10}$, $R^4$ and $R^9$, $R^{5a}$ and $R^{8a}$, $R^{5b}$ and $R^{8b}$, $R^6$ and $R^7$ as well as $A^1$ and $A^2$. More preferably, the substituents are the same in any of the respective combinations.

In one embodiment of the present invention that n is 1 in the polyacene derivatives represented by the above formula (I), $A^1$ and $A^2$ may be an alkoxycarbonyl group, and $R^1$, $R^2$, $R^4$ and $R^9$ may be an alkyl or aryl group. Also when n is 1, $A^1$, $A^2$, $R^1$, $R^2$, $R^4$ and $R^9$ may be an alkyl or aryl group. Further when n is 1, $A^1$ and $A^2$ are a halogen atom and $R^3$, $R^5$, $R^6$, $R^7$, $R^8$ and $R^{10}$ may be an alkyl or aryl group.

In one embodiment of the present invention, when the polyacene derivatives are the pentacene derivatives represented by the formula (Ia) above, $A^1$ and $A^2$ may be an alkoxycarbonyl group and $R^1$, $R^2$, $R^4$, $R^{5b}$, $R^6$, $R^7$, $R^{8b}$ and $R^9$ may be an alkyl or aryl group. Also, when the polyacene derivatives described above are the pentacene derivatives represented by the formula (Ia) above, $A^1$, $A^2$, $R^1$, $R^2$, $R^4$, $R^{5b}$, $R^6$, $R^7$, $R^{8b}$ and $R^9$ may be an alkyl or aryl group. Furthermore, when the polyacene derivatives described above are the pentacene derivatives represented by the formula (Ia) above, $A^1$ and $A^2$ may be a halogen atom and $R^3$, $R^{5a}$, $R^{8a}$ and $R^{10}$ may be an alkyl or aryl group.

Specific examples of the polyacene derivatives of general formula (I), which are the materials for organic electroluminescent elements of the present invention, are listed below.

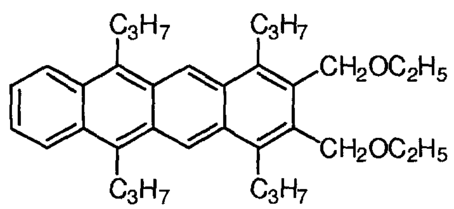

(Compound 1)

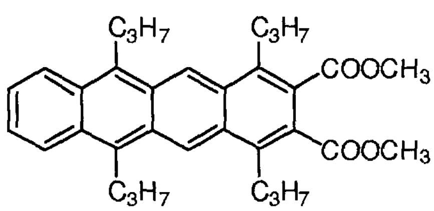

(Compound 2)

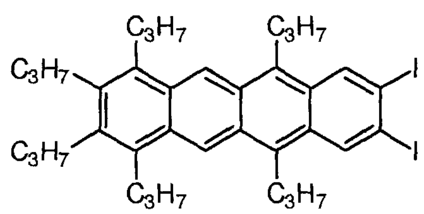

(Compound 3)

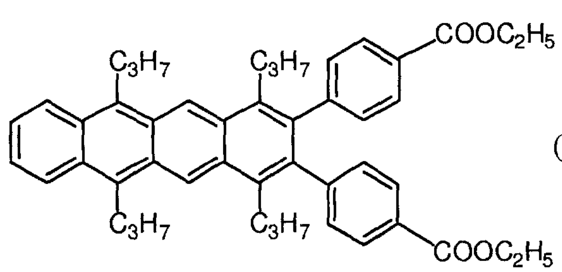

(Compound 4)

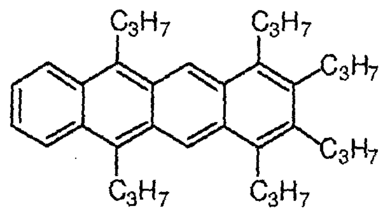

(Compound 5)

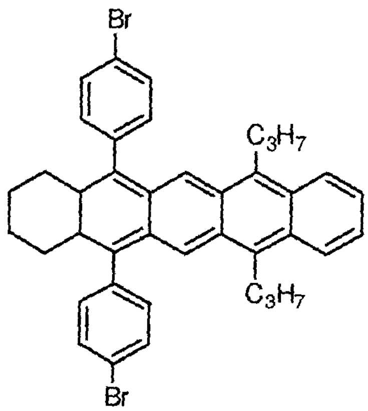

(Compound 6)

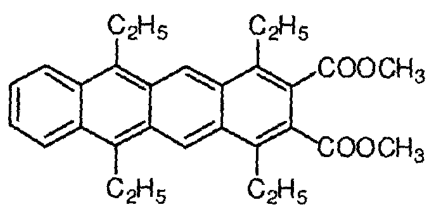

(Compound 7)

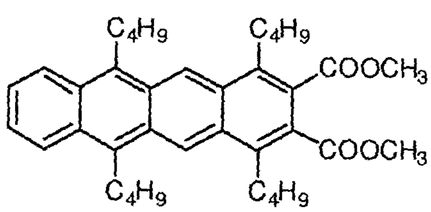

(Compound 8)

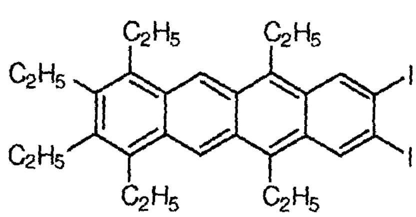

(Compound 9)

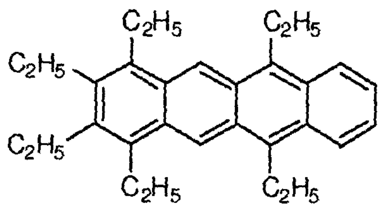

(Compound 10)

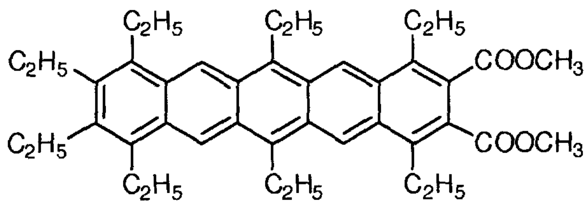

(Compound 11)

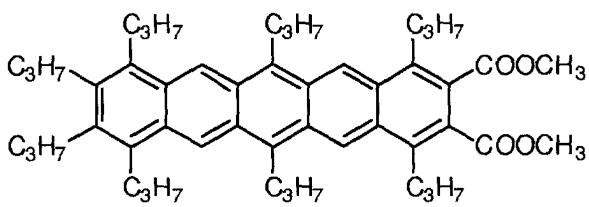

(Compound 12)

As a process of producing the polyacene derivatives of general formula (I), which are the materials for organic electroluminescent elements of the present invention, there can be, for example, a process of producing the polyacene derivatives represented by the formula (I) above, which comprises aromatizing the hydrocarbon condensed rings represented by formula (II) below:

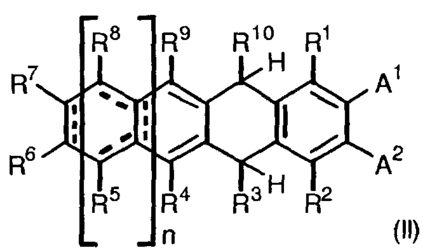

(II)

[wherein $R^1$, $R^2$, $R^3$, $R^4$, $R^5$, $R^6$, $R^7$, $R^8$, $R^9$, $R^{10}$, $A^1$, $A^2$, and n have the same significance as defined above; and the bond shown by formula below:

------

represents a single bond or a double bond], in the presence of a dehydrogenation reagent.

The hydrocarbon condensed rings represented by formula (II) above include, e.g., the following hydrocarbon condensed rings represented by (IIa), (IIb) and (IIc), depending on the kind of bonding:

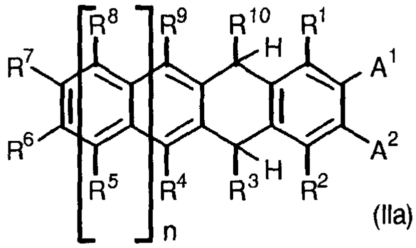

(IIa)

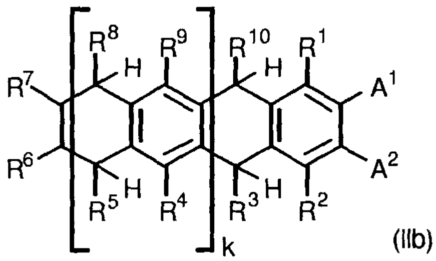

(IIb)

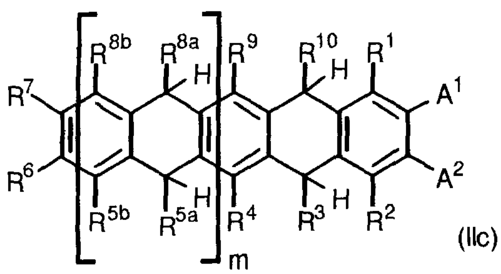

(IIc)

[wherein $R^1$, $R^2$, $R^3$, $R^4$, $R^5$, $R^{5a}$, $R^{5b}$, $R^6$, $R^7$, $R^8$, $R^{8a}$, $R^{8b}$, $R^9$, $R^{10}$, $A^1$, $A^2$, and n have the same significance as described above].

When n is an odd number and the hydrocarbon condensed rings represented by formula (II) described above are those represented by formula (IIb) above, k is an integer shown by (n+1)/2, and when n is an even number and the hydrocarbon condensed rings represented by formula (II) described above are those represented by formula (IIc) above, m is an integer shown by n/2.

In the hydrocarbon condensed rings represented by formula (IIa), it means that one ring is aromatized. On the other hand, in the hydrocarbon condensed rings represented by formula (IIb) and formula (IIc), it means that two or more rings are aromatized.

As a matter of course, the hydrocarbon condensed rings represented by formula (II) further include the cases wherein the rings in a repeating unit being an aromatic ring and a non-aromatic ring are repeated at random.

In the process of producing the polyacene derivatives represented by the formula (I) described above, the dehydrogenation reagent is preferably a combination of a lithium dopant and a lithium-removing reagent. It is preferred to add the lithium dopant first to the hydrocarbon condensed rings followed by adding the lithium-removing reagent thereto.

This scheme is illustratively shown for the cases of the hydrocarbon condensed rings represented by formula (IIa), (IIb) and (IIc) below.

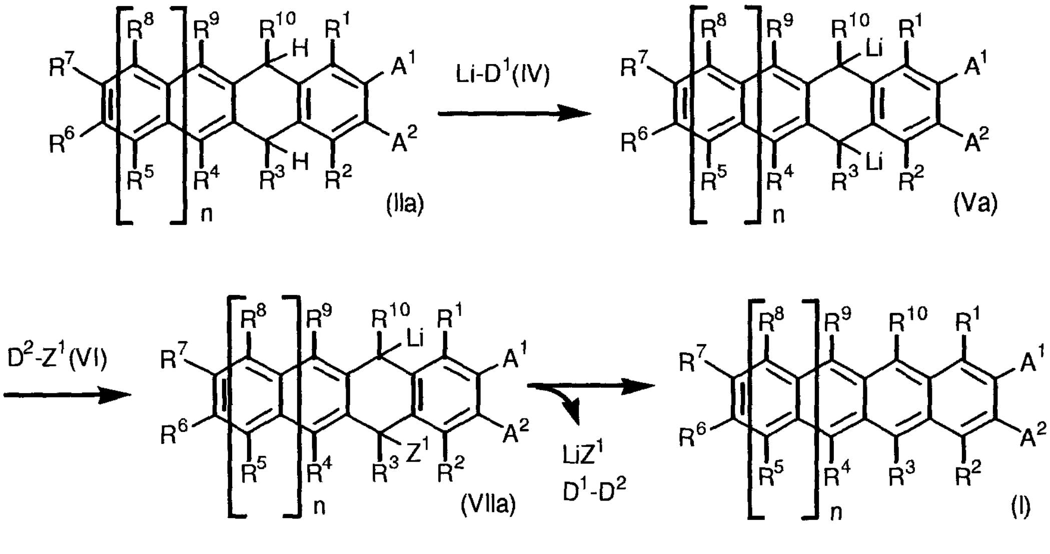


[wherein $R^1$, $R^2$, $R^3$, $R^4$, $R^5$, $R^6$, $R^7$, $R^8$, $R^9$, $R^{10}$, $A^1$, $A^2$, and n have the same significance as described above; $D^1$ represents a nucleophilic group such as a $C_1$-$C_6$ alkyl group, etc.; $D^2$ represents a $C_1$-$C_{20}$ hydrocarbon group such as a $C_1$-$C_6$ alkyl group, etc.; and $Z^1$ represents an leaving group such as a halogen atom, etc.].

In this reaction, $R^3$ and $R^{10}$ in the formula (IIa) are preferably hydrogen atoms, in view of easy synthesis of the polyacene derivatives.

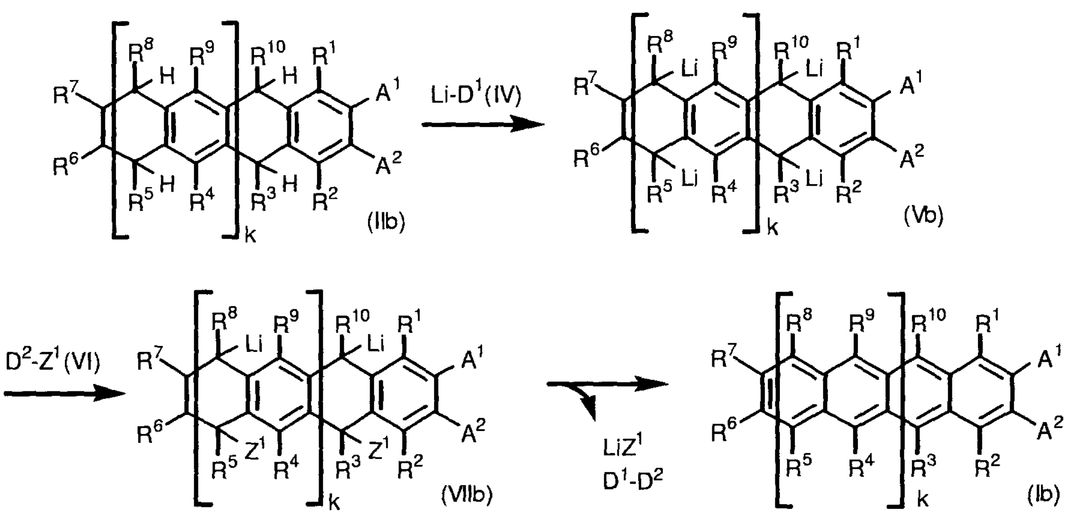


[wherein $R^1$, $R^2$, $R^3$, $R^4$, $R^5$, $R^6$, $R^7$, $R^8$, $R^9$, $R^{10}$, $A^1$, $A^2$, and k have the same significance as described above; $D^1$ represents a nucleophilic group such as a $C_1$-$C_6$ alkyl group, etc.; $D^2$ represents a $C_1$-$C_{20}$ hydrocarbon group such as a $C_1$-$C_6$ alkyl group, etc.; and $Z^1$ represents an leaving group such as a halogen atom, etc.].

In this reaction, $R^3$, $R^5$, $R^8$ and $R^{10}$ in the formula (IIb) are preferably hydrogen atoms, in view of easy synthesis of the polyacene derivatives.

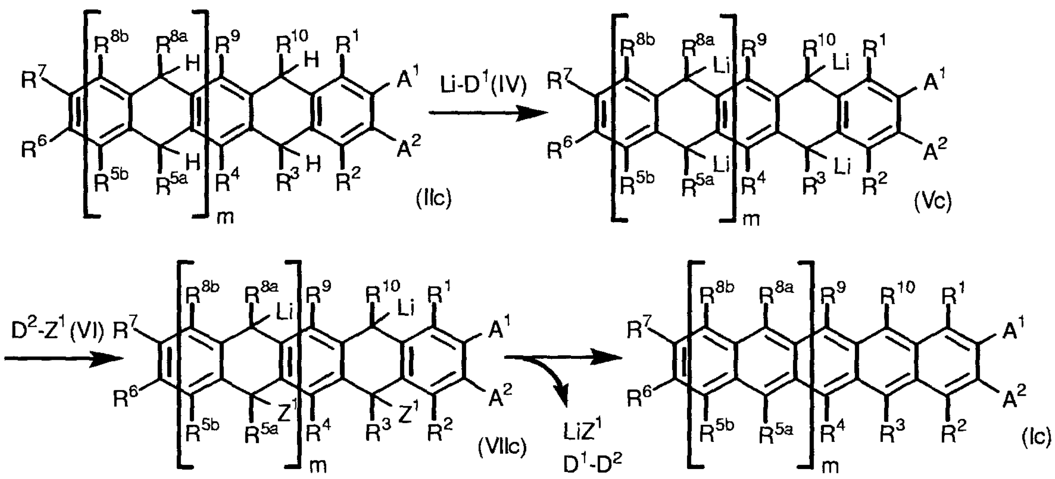


(IIc) (Vc) (VIIc) (Ic)

[wherein $R^1$, $R^2$, $R^3$, $R^4$, $R^5$, $R^6$, $R^7$, $R^8$, $R^9$, $R^{10}$, $A^1$, $A^2$, and m have the same significance as described above; $D^1$ represents a nucleophilic group such as a $C_1$-$C_6$ alkyl group, etc.; $D^2$ represents a $C_1$-$C_{20}$ hydrocarbon group such as a $C_1$-$C_6$ alkyl group, etc.; and $Z^1$ represents an leaving group such as a halogen atom, etc.].

In this reaction, $R^3$, $R^{5a}$, $R^{8a}$, $R^{10}$ in the formula (IIc) are preferably hydrogen atoms, in view of easy synthesis of the polyacene derivatives.

In the schemes described above, the hydrocarbon condensed rings represented by formulae (IIa), (IIb) or (IIc) are employed, for the sake of explanation to clarify the carbon atoms on which the lithium dopant (IV) shown by Li-$D^1$ acts. It goes without saying that the dehydrogenation reagent in the combination of the lithium dopant and the lithium-removing reagent is widely applicable to the hydrocarbon condensed rings shown by formula (II) described above.

The lithium dopant (IV) is reacted with the hydrocarbon condensed rings represented by the formulae (IIa), (IIb) and (IIc) to obtain the lithium-doped hydrocarbon condensed rings represented by formulae (Va), (Vb) and (Vc), respectively. Preferred lithium dopants include a $C_1$-$C_{20}$ hydrocarbon lithium such as an alkyl lithium, an aryl lithium, etc. For example, a $C_1$-$C_6$ alkyl lithium such as butyl lithium, etc., a $C_6$-$C_{20}$ aryl lithium such as phenyl lithium, etc. are preferably used.

It is preferred that an activator of the lithium dopant co-exists together with the lithium dopant (IV). As the activator, tertiary amines are preferred and for example, N,N,N',N'-tetraalkylalkylenediamines such as N,N,N',N'-tetramethylethylenediamine (TMEDA) are employed. It is likely that the alkyl lithium would be present in a solution as an oligomer like a tetramer. When a tertiary amine is co-present, it is assumed that the nitrogen atom of the amine would be coordinated on the lithium atom of the alkyl lithium to cleave the oligomer structure, whereby the lithium atom in the alkyl lithium would be exposed to the solution to improve the reactivity.

A preferred solvent is an organic solvent. In particular, a non-polar organic solvent is employed. For example, an alkane such as hexane, etc. and an aromatic compound such as benzene, etc. are preferred.

A preferred reaction temperature is from 0°C to 200°C, more preferably 20°C to 100°C, and most preferably 30°C to 80°C.

When the lithium-removing reagent (VI) is reacted with the hydrocarbon condensed rings shown by formulae (Va), (Vb) and (Vc), it is speculated that the intermediates represented by formulae (VIIa), (VIIb) and (VIIc), respectively, will be formed. The intermediates are decomposed to give the polyacene derivatives represented by formula (I), (Ib) or (Ic).

As the lithium-removing reagent (VI), for example, alkyl halides are advantageously used. Preferred examples of the alkyl halides are alkyl halides having 6 or less carbon atoms, such as methyl iodide, ethyl bromide, etc.

Where the lithium dopant (IV) and the lithium-removing reagent (VI) having less carbon atoms, such as butyl lithium and methyl iodide are used as the lithium dopant (IV) and the lithium-removing reagent (VI), respectively, lithium iodide and hexane will be split off. Hexane can be removed at the same time when the solvent is removed. Lithium iodide can be removed by washing the resulting reaction mixture with water. Thus, the combination of the lithium dopant and the lithium-removing reagent renders purification of the reaction mixture extremely easy and is desirable.

When a large number of hydrogen atoms are introduced on $R^1$, $R^2$, $R^3$, $R^4$, $R^5$, $R^6$, $R^7$, $R^8$, $R^9$, $R^{10}$, $A^1$ and $A^2$, e.g., when at least 8 of these groups are hydrogen atoms, the yield of the polyacene derivative represented by formula (I) based on the hydrocarbon condensed rings represented by formula (IIa) is approximately 50%. On the other hand, when at least 6, especially 8 or more groups other than hydrogen atom are introduced on $R^1$, $R^2$, $R^3$, $R^4$, $R^5$, $R^6$, $R^7$, $R^8$, $R^9$, $R^{10}$, $A^1$ and $A^2$, the yield tends to increase. For example, the yield occasionally reaches 90% or more,

or sometimes becomes 95% or more.

Furthermore, in the process of producing the polyacene derivatives of the general formula (I) above, the dehydrogenation reagent described above is preferably compounds represented by formula (III) below:

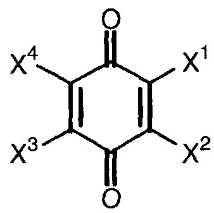

(III)

[wherein $X^1$, $X^2$, $X^3$ and $X^4$, which may be the same or different, independently represents a halogen atom or cyano group].

The quinones represented by the formula (III) above are reacted with the compounds represented by the formula (II) above to convert into 1,4-dihydroxy-cyclohexane derivatives.

In the quinones represented by the formula (III) above, the halogen atom is preferably chlorine atom, bromine atom or iodine atom, more preferably chlorine atom or bromine atom, and most preferably chlorine atom.

For example, all of $X^1$, $X^2$, $X^3$ and $X^4$ may be chlorine atoms. That is, the quinone may be chloranil. Alternatively, $X^1$ and $X^2$ may be cyano group, and $X^3$ and $X^4$ may be chlorine atoms. That is, it may be 2,3-dichloro-5,6-dicyanoquinone. Alternatively again, $X^1$, $X^2$, $X^3$ and $X^4$ may all be cyano groups. That is, it may be 2,3,5,6-tetracyanoquinone.

When the quinones represented by the formula (III) above are used, the quinones represented by the formula (III) above may occasionally undergo Diels-Alder reaction with the polyacene derivative products to produce by-products. If desired, the by-products are removed by column chromatography, etc.

In order to prevent the production of such by-products, the quinones represented by the formula (III) above are used preferably in 0.9 to 1.2 equivalents, more preferably 0.9 to 1.15 equivalents, and most preferably 0.95 to 1.05 equivalents, based on the compounds represented by formula (II) described above.

As the solvent, an organic solvent is preferred, and an aromatic compound such as benzene, etc. is particularly preferred.

The reaction temperature is preferably from -80°C to 200°C, more preferably from 0°C to 100°C, and most preferably from 10°C to 80°C. If desired, the reaction may be carried out under light shielding.

Furthermore, in the process of producing the polyacene derivatives of the general formula (I) above, it is preferred that the dehydrogenation reagent described above includes palladium. For example, palladium carried on carbon such as activated carbon, which is commercially available as so-called palladium carbon, may preferably be employed. Pd/C is a catalyst that has been widely used for dehydrogenation, and can be used in the present invention as in a conventional manner. The reaction temperature is, e.g., from 200 to 500°C. Of course, the reaction temperature may appropriately be set, depending upon various conditions including starting materials, etc.

The hydrocarbon condensed rings can be obtained, e.g., by the following scheme.

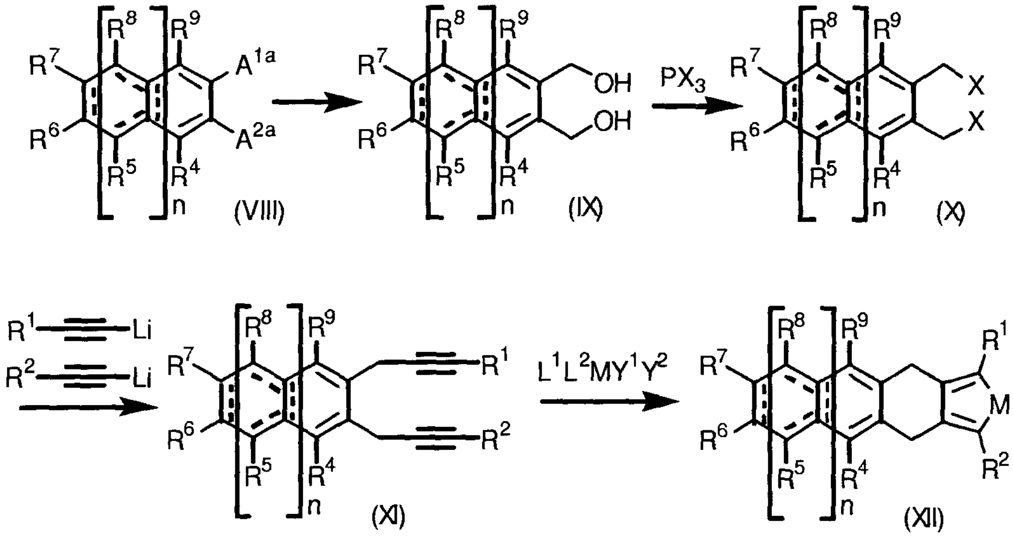


(VIII) (IX) (X) (XI) (XII)

[wherein $R^1$, $R^2$, $R^4$, $R^5$, $R^6$, $R^7$, $R^8$, $R^9$ and n have the same significance as defined above; $A^{1a}$ and $A^{2a}$, which may be the same or different, independently represents a $C_6$-$C_{40}$ alkoxycarbonyl group which may optionally be substituted with a substituent, including a halogen atom, or a $C_6$-$C_{40}$ aryloxycarbonyl group which may optionally be substituted a substituent, including a halogen atom; and X is an leaving group such as a halogen atom, etc.; the bond represented by formula below is a single bond or a double bond;

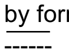

M represents a metal belonging to the Group III to Group V or a lanthanide metal;

$L^1$ and $L^2$, which may be the same or different, independently represents an anionic ligand, provided that $L^1$ and $L^2$ may be cross-bridged with each other; and,

$Y^1$ and $Y^2$, which may be the same or different, independently represents an leaving group].

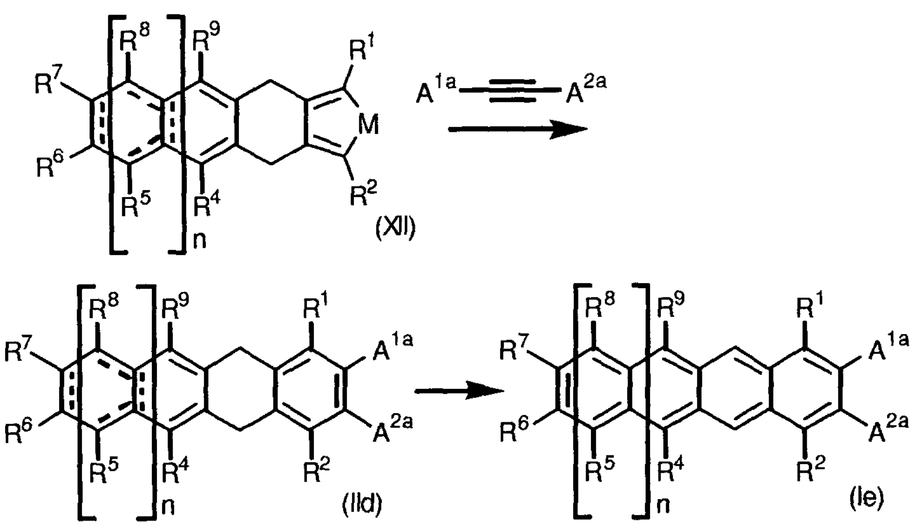

(XII) (IId) (Ie)

Next, the organic electroluminescent element is described. An organic electroluminescent element normally comprises a pair of electrodes and at least one luminescent layer containing at least one luminescent component, sandwiched between the electrodes. Taking into account the respective functional levels of the hole injection and hole transport of a compound used in the luminescent layer, injection of electrons and transport of electrons, a hole injection transport layer containing a hole injection transport component and/or an electron injection transport layer containing an electron injection transport component can also be provided. For example, in case that the compound used in the luminescent layer is good in hole injection function and hole transporting function and/or electron injection function and electron transport function, the luminescent layer can be constructed as an element that the luminescent layer serves

as the hole injection transport layer and/or the electron injection transport layer. Also, the element may also be a structure type where both the hole injection transport layer and the electron injection transport layer are not provided (a single-layered type element), as the case may be. Furthermore, each of the hole injection transport layer, the electron injection transport layer and the luminescent layer may take a single-layered structure or a multi-layered structure. The hole injection transport layer and the electron injection transport layer may also be so constructed to have a layer having the injection function and a layer having the transporting function separately in the respective layers.

In the organic electroluminescent element of the present invention, the polyacene derivatives represented by the general formula (I) are preferably used as a component for hole injection transporting, light emission or electron injection transporting, more preferably as a component for hole injection transporting or light emission. In the organic electroluminescent element of the present invention, the polyacene derivatives of the present invention may be used alone or with more than one.

The configuration of the organic electroluminescent element of the present invention is not particularly limited but includes, for example, (A) anode/hole injection transport layer/luminescent layer/electron injection transport layer/anode type element (FIG. 1), (B) anode/hole injection transport layer/luminescent layer/anode type element (FIG. 2), (C) anode/luminescent layer/electron injection transport layer/anode type element (FIG. 3), (D) anode/luminescent layer/anode type element (FIG. 4), etc. In addition, the element may also be in the configuration of (E) anode/hole injection transport layer/electron injection transport layer/luminescent layer/electron injection transport layer/anode type element (FIG. 5), in which the luminescent layer is sandwiched between the electron injection transport layers. In the element configuration of type (D), the luminescent component is certainly sandwiched between a pair of electrodes in a single-layered structure, and additionally the element includes, for example, (F) the type in which the luminescent component is sandwiched between a pair of electrodes in a single- layered structure wherein the hole injection transporting component, the luminescent component and the electron injection transporting component are mixed (FIG. 6), (G) the type in which the luminescent component is sandwiched between a pair of electrodes in a single-layered structure wherein the hole injection transporting component and the luminescent component are mixed (FIG. 7), and (H) the type in which the luminescent component is sandwiched between a pair of electrodes in a single- layered structure wherein the luminescent component and the electron injection transporting component are mixed (FIG. 8).

The organic electroluminescent element of the present invention is not limited to these element configurations but in each type of the element, the hole injection transport layer, the luminescent layer and the electron injection transport layer can be provided in a plurality of layers. Furthermore, in each type of the element, a layer of a mixture of the hole injection transporting component and the luminescent component is provided between the hole injection transport layer and the luminescent layer, and/or between the luminescent layer and the electron injection transport layer, a layer of a mixture of the luminescent component and the electron injection transporting component can be provided.

Preferred configurations of the organic electroluminescent element are the elements of type (A), type (B), type (C), type (E), type (F), type (G) and type (H), more preferably the elements of type (A), type (B), type (C), type (F) and type (G). Referring to, e.g., (A) anode/hole injection transport layer/luminescent layer/electron injection transport layer/cathode type element shown in (FIG. 1), the organic electroluminescent element of the present invention is explained. In (FIG. 1), 1, 2, 3, 4, 5, 6 and 7 denotes a substrate, an anode, a hole injection transport layer, a luminescent layer, an electron injection transport layer, a cathode and electric source, respectively.

The organic electroluminescent element of the present invention is preferably supported on the substrate 1. The substrate is not particularly limited but preferably transparent or opaque, and includes, for example, a glass plate, a transparent plastic sheet (e.g., a sheet of polyester, polycarbonate, polysulfone, polymethyl methacrylate, polypropylene, polyethylene, etc.), an opaque plastic sheet, quartz, transparent ceramics, or a mixture thereof in a composite sheet. In addition, the substrate may also be used in combination with, e.g., a color filter film, a color conversion film or a dielectric reflecting film to control the emitting color.

For the anode 2, metals, alloys or electroconductive compounds having a relatively large work function are preferably used as the electrode material. Examples of the electrode material used for the anode include gold, platinum, silver, copper, cobalt, nickel, palladium, vanadium, tungsten, tin oxide, zinc oxide, ITO (indium tin oxide), polythiophene, polypyrrole, etc. These electrode materials may be used solely or in a mixture thereof. The anode can be formed on the substrate by treating these electrode materials using methods, e.g., a vapor deposition method, sputtering method, etc. The anode may be in a single-layered structure or a multi-layered structure. Sheet electric resistance of the anode is set out preferably in several hundreds O/? or less, more preferably in approximately 5 to 50 O/? . The thickness of the anode may vary depending on materials used for the electrode material and is generally set in approximately 5 to 1000 nm, preferably approximately 10 to 500 nm.

The hole injection transport layer 3 is a layer which contains the compound having the function to make the hole injection from the anode easy and the function to transport the injected holes. The hole injection transport layer can be formed using at least one of the polyacene derivative of the present invention and/or other compounds having the hole injection transport function (for example, phthalocyanine derivatives, triarylmethane derivatives, triarylamine

derivatives, oxazole derivatives, hydrazone derivatives, stilbene derivatives, pyrazoline derivatives, polysilane derivatives, polyphenylenevinylene and its derivatives, polythiophene and its derivatives, poly-N-vinylcarbazole derivatives, etc.). The compound having the hole injection transport function may be used alone or with more than one.

As the other compounds having the hole injection transport function used in the present invention, more preferred are triarylamine derivatives (e.g., 4,4'-bis[N-phenyl-N-(4"-methylphenyl)amino]biphenyl, 4,4'-bis[N-phenyl-N-(3"-methylphenyl)amino]biphenyl, 4,4'-bis[N-phenyl-N-(3"-methoxyphenyl)amino]biphenyl, 4,4'-bis[N-phenyl-N-(1"-naphthyl)amino]biphenyl, 3,3'-dimethyl-4,4'-bis[N-phenyl-N-(3"-methylphenyl)amino]biphenyl, 1,1-bis[4'-[ N, N-di-(4"-methylphenyl)amino] phenyl]cyclohexane, 9,10-bis[N-(4'-methylphenyl)-N-(4"-n-butylphenyl)amino]phenanthrene, 3,8-bis(N,N-diphenylamino)-6-phenylphenanthridine, 4-methyl-N,N-bis[4",4"'-bis[ N',N'-di-(4-methylphenyl) amino] biphenyl-4-yl]aniline, N,N'-bis[4-(diphenylamino)phenyl]-N,N'-diphenyl-1,3-diaminobenzene, N,N'-bis[4-(diphenylamino)phenyl]-N,N'-diphenyl-1,4-diaminobenzene, 5,5"-bis[4-(bis[4-methylphenyl] amino)phenyl]-2,2':5',2"-terthiophene, 1,3,5-tris(diphenylamino)benzene, 4,4',4"-tris(N-carbazolyl)triphenylamine, 4,4',4"-tris[N-(3"'-methylphenyl) -N-phenylamino)triphenylamine, 4,4',4"-tris[N,N-bis(4"'-tert-butylbiphenyl-4""-yl)amino]triphenylamine, 1,3,5-tris [N-(4'-diphenylaminophenyl)-N-phenylamino]benzene, etc.), polythiophene and its derivatives and poly-N-vinylcarbazole derivatives.

When the polyacene derivative of the present invention and the other compound having the hole injection transport function are used in combination, the ratio of the polyacene derivative of the present invention to the hole injection transport layer is preferably 0.1% by weight or more, more preferably about 0.1-99.9% by weight, much more preferably about 1-99% by weight and most preferably about 5-95% by weight.

The luminescent layer 4 is a layer which contains the compound having the functions to inject holes and electrons, transport them and generate excitons through recombination of the holes and electrons. The luminescent layer can be formed using at least one of the polyacene derivatives of the present invention and/or other compounds having the light-emitting function (e.g., acridone derivatives, quinacridone derivatives, diketo-pyrrolopyrrole derivatives, polycyclic aromatic compounds [e.g., rubrene, anthracene, tetracene, pyrene, perylene, chrysene, decacyclene, coronene, tetraphenylcyclopentadiene, pentaphenylcyclopentadiene, 9,10-diphenylanthracene, 9,10-bis(phenylethynyl) anthracene, 1,4-bis(9'-ethynylanthracenyl)benzene, 4,4'-bis(9"-ethynylanthracenyl)biphenyl], triarylamine derivatives [e.g., the compounds described above as the compounds having the hole injection transport function can be listed], organic metal complexes [e.g., tris(8-quinolinolato)aluminum, bis(10-benzo[h]quinolinolato)beryllium, 2- (2'-hydroxyphenyl)benzoxazole zinc salt, 2-(2'-hydroxyphenyl)benzothiazole zinc salt, 4-hydroxyacridine zinc salt, 3-hydroxyflavone zinc salt, 5-hydroxyflavone beryllium salt, 5-hydroxflavone aluminum salt], stilbene derivatives [e.g., 1,1,4,4-tetraphenyl-1,3-butadiene, 4,4'-bis(2,2-diphenylvinyl)biphenyl, 4,4'-bis[ (1,1,2-triphenyl)ethenyl] biphenyl], coumarin derivatives [e.g., Coumarin 1, Coumarin 6, Coumarin 7, Coumarin 30, Coumarin 106, Coumarin 138, Coumarin 151, Coumarin 152, Coumarin 153, Coumarin 307, Coumarin 311, Coumarin 314, Coumarin 334, Coumarin 338, Coumarin 343, Coumarin 500], pyrane derivatives [e.g., DCM1, DCM2], oxazone derivatives [e.g., Nile red], benzothiazole derivatives, benzoxazole derivatives, benzimidazole derivatives, pyrazine derivatives, cinnamate derivatives, poly-N-vinylcarbazole and its derivatives, polythiophene and its derivatives, polyphenylene and its derivatives, polyfluorene and its derivatives, polyphenylenevinylene and its derivatives, polybiphenylenevinylene and its derivatives, polyterphenylenevinylene and its derivatives, polynaphthylenevinylene and its derivatives, polythienylenevinylene and its derivatives, etc.).

In the organic electroluminescent element of the present invention, the luminescent layer preferably contains the polyacene derivative of the present invention. When the polyacene derivative of the present invention and the other compound having the light-emitting function are used in combination, the luminescent layer is so prepared that the ratio of the polyacene derivative in the luminescent layer is preferably in about 0.001-99.999% by weight, more preferably in about 0.01-99.99% by weight and most preferably in about 0.1-99.9% by weight.

As the other compounds having the light-emitting function used in the present invention, polycyclic aromatic compounds, luminescent organic metal complexes and triarylamine derivatives are more preferred. For example, the luminescent layer can be formed of a host compound and a guest compound (dopant), as described in J. Appl. Phys., 65, 3610 (1989) or Japanese Laid-Open Publication No. 5-214332. The luminescent layer can be formed by using the polyacene derivative of the present invention as a host compound. The polyacene derivative can also be used as a guest compound to form the luminescent layer. In case that the polyacene derivative of the present invention is used as the host compound to form the luminescent layer, the other compounds having the light-emitting function described above can be used as the guest compound and polycyclic aromatic compounds are preferred, among others. In this case, the other compound having the light-emitting function is used in preferably about 0.001-40% by weight, more preferably about 0.01-30% by weight and most preferably about 0.1-20% by weight, based on the polyacene derivative of the present invention.

The polycyclic aromatic compounds, which are used in combination with the polyacene derivatives of the present invention, are not particularly limited but examples include rubrene, anthracene, tetracene, pyrene, perylene, chrysene, decacyclene, coronene, tetraphenylcyclopentadiene, pentaphenylcyclopentadiene, 9,10-diphenylanthracene, 9,10-bis(phenylethynyl)anthracene, 1,4-bis(9'-ethynylanthracenyl)benzene, 4,4'-bis(9'-ethynylanthracenyl)biphenyl, etc. Certainly, the polycyclic aromatic compounds can be used solely or in combination of plural kinds.

Where the polyacene derivative of the present invention is used as the guest compound to form the luminescent layer, for example, the other compound having the light-emitting function described above can be used as the host compound and, for example, the luminescent organic metal complex or triarylamine derivative is more preferred. In this case, the polyacene derivative of the present invention is used in preferably about 0.001-40% by weight, more preferably about 0.01-30% by weight and most preferably about 0.1-20% by weight, based on the luminescent organic metal complex or triarylamine derivative.

The luminescent organic metal complexes, which are used in combination with the polyacene derivatives of the present invention, are not particularly limited but luminescent organic aluminum complexes are preferred, and more preferred are luminescent organic aluminum complexes having a substituted or unsubstituted 8-quinolinolato ligand. As the preferred luminescent organic metal complexes, there are, for example, luminescent organic aluminum complexes represented by general formulae (a) through (c).

$$(Q)\ 3 - Al \qquad (a)$$

[wherein Q represents a substituted or unsubstituted 8-quinolinolato ligand]

$$(Q)\ 2 - Al - O - L \qquad (b)$$

[wherein Q represents a substituted 8-quinolinolato ligand, O-L is a phenolato ligand, and L represents a $C_{6-24}$ hydrocarbon group containing the phenyl moiety]

$$(Q)\ 2 - Al - O - Al - (Q)\ 2 \qquad (c)$$

[wherein Q represents a substituted 8-quinolinolato ligand].

Specific examples of the luminescent organic metal complex include tris(8-quinolinolato)aluminum, tris(4-methyl-8-quinolinolato)aluminum, tris(5-methyl-8-quinolinolato)aluminum, tris(3,4-dimethyl-8-quinolinolato)aluminum, tris(4,5-dimethyl-8-quinolinolato)aluminum, tris(4,6-dimethyl-8-quinolinolato)aluminum, bis(2-methyl-8-quinolinolato)(phenolato)aluminum, bis(2-methyl-8-quinolinolato)(2-methylphenolato)aluminum, bis(2-methyl-8-quinolinolato)(3-methylphenolato)aluminum, bis(2-methyl-8-quinolinolato)(4-methylphenolato)aluminum, bis(2-methyl-8-quinolinolato)(2-phenylphenolato)aluminum, bis(2-methyl-8-quinolinolato)(3-phenylphenolato)aluminum, bis(2-methyl-8-quinolinolato)(4-phenylphenolato)aluminum, bis(2-methyl-8-quinolinolato)(2,3-dimethylphenolato)aluminum, bis(2-methyl-8-quinolinolato)(2,6-dimethylphenolato)aluminum, bis(2-methyl-8-quinolinolato)(3,4-dimethylphenolato)aluminum, bis(2-methyl-8-quinolinolato)(3,5-dimethylphenolato)aluminum, bis(2-methyl-8-quinolinolato)(3,5-di-tert-butylphenolato)aluminum, bis(2-methyl-8-quinolinolato)(2,6-diphenylphenolato)aluminum, bis(2-methyl-8-quinolinolato)(2,4,6-triphenylphenolato)aluminum, bis(2-methyl-8-quinolinolato)(2,4,6-trimethylphenolato)aluminum, bis(2-methyl-8-quinolinolato)(2,4,5,6-tetramethylphenolato)aluminum, bis(2-methyl-8-quinolinolato)(1-naphtholate)aluminum, bis(2-methyl-8-quinolinolato)(2- naphtholate)aluminum, bis(2,4-dimethyl-8-quinolinolato)(2-phenylphenolato)aluminum, bis(2,4-dimethyl-8-quinolinolato)(3-phenylphenolato)aluminum, bis(2,4-dimethyl-8-quinolinolato)(4-phenylphenolato)aluminum, bis(2,4-dimethyl-8-quinolinolato)(3,5-dimethylphenolato)aluminum, bis(2,4-dimethyl-8-quinolinolato)(3,5-di-tert-butylphenolato)aluminum, bis(2-methyl-8-quinolinolato)aluminum-μ-oxo-bis(2-methyl-8-quinolinolato)aluminum, bis(2,4-dimethyl-8-quinolinolato)aluminum-μ-oxo-bis(2,4-dimethyl-8-quinolinolato)aluminum, bis(2-methyl-4-ethyl-8-quinolinolato)aluminum-μ-oxo-bis(2-methyl-4-ethyl-8-quinolinolato)aluminum, bis(2- methyl-4- methoxy-8-quinolinolato)aluminum-μ-oxo-bis(2-methyl-4-methoxy-8-quinolinolato)aluminum, bis(2-methyl-5-cyano-8-quinolinolato)aluminum-μ-oxo-bis(2-methyl-5-cyano-8-quinolinolato)aluminum, bis(2-methyl-5-trifluoromethyl-8-quinolinolato)aluminum-μ-oxo-bis(2-methyl-5-trifluoromethyl-8-quinolinolato)aluminum, etc. Of course, the luminescent organic metal complex may be used alone or with more than one.

The electron injection transport layer 5 is a layer which contains the compound having the functions to render injection of the electrons through the cathode and transport the injected electrons. The electron injection transport layer can be formed using at least one of the polyacene derivatives of the present invention and/or the other compounds having the electron injection transport function (for example, organic metal complexes [e.g., tris(8-quinolinolato)aluminum, bis(10-benzo[h]quinolinolato)beryllium, 5-hydroxyflavone beryllium salt, 5-hydroxyflavone aluminum salt], oxadiazole derivatives [e.g., 1,3-bis[ 5'-(p-tert-butylphenyl)-1,3,4-oxadiazol-2'-yl] benzene], triazole derivatives [e.g. 3-(4'-tert-butylphenyl)-4-phenyl-5-(4''-biphenyl)-1,2,4-triazole], triazine derivatives, perylene derivatives, quinoline derivatives, quinoxaline derivatives, diphenylquinone derivatives, nitro-substituted fluorenone derivatives, thiopyrane dioxide derivatives, etc.).

In the case that the polyacene derivative of the present invention and the other compound having the electron injection transport function are used in combination, the polyacene derivative of the present invention in the electron injection transport layer is adjusted in the ratio of preferably about 0.1-40% by weight. In the present invention, the electron injection transport layer is formed preferably by using the polyacene derivatives of the present invention in combination with the organic metal complexes [for example, the compounds represented by formulae (a) to (c) described above].

For the cathode 6, metals, alloys or electroconductive compounds having a relatively small work function are preferably used as the electrode material. Examples of the electrode material used for the cathode include lithium, lithium-indium alloy, sodium, sodium-potassium alloys, calcium, magnesium, magnesium-silver alloys, magnesium-indium alloys, indium, ruthenium, titanium, manganese, yttrium, aluminum, aluminum-lithium alloys, aluminum-calcium alloys, aluminum- magnesium alloys, graphite thin film, etc. These electrode materials may be used alone or with one or more.

The cathode can be formed on the electron injection transport layer by means of, e.g., a vapor deposition method, sputtering method, ionized deposition method, ion plating method, cluster ion beam method, etc. Furthermore, the cathode may be a single-layered structure or a multi-layered structure. Sheet electric resistance of the cathode is set preferably in several hundreds O/? or less. The thickness of the cathode may vary depending on materials used for the electrode material and is generally set in approximately 5 to 1000 nm, preferably approximately 10 to 500 nm. To take out the emission of the organic electroluminescent element efficiently, either one of the electrodes for the anode or cathode is preferably transparent or opaque. In general, it is more preferred to choose the material of the anode and control its thickness to have not less than 70% transmittance of the emitted light.

In the organic electroluminescent element of the present invention, a singlet oxygen quencher may be contained in at least one layer of the element. The singlet oxygen quencher is not particularly limited but includes, e.g., rubrene, a nickel complex, diphenylisobenzofurane, etc., with rubrene being particularly preferred. The layer in which the singlet oxygen quencher is contained is not particularly limited but preferably the luminescent layer or the hole injection transport layer, more preferably the hole injection transport layer. Where the singlet oxygen quencher is incorporated, e.g., in the hole injection transport layer, the quencher may be incorporated uniformly in the hole injection transport layer, or contained near the layer adjacent to the hole injection transport layer (e.g., the luminescent layer, the electron injection transport layer having the light-emitting function). The amount of the singlet oxygen quencher contained is 0.01-50% by weight, preferably 0.05-30% by weight, more preferably 0.1-20% by weight, based on the total amount of the layer containing the quencher (e.g., the hole injection transport layer).

It is not particularly limited how to form the hole injection transport layer, the luminescent layer and the electron injection transport layer. These layers can be prepared by forming thin films by means of, e.g., a vacuum deposition method, ionized deposition method or solution-coating process (e.g., spin coating, casting, dip coating, bar coating, roll coating, Langmuir-Blodgett technique, ink jet process, etc.). In the case that the respective layers are formed by the vacuum deposition method, conditions for the vacuum deposition are not particularly limited but the deposition is preferably carried out in vacuum of about $10^{-5}$ Torr or less at a boat temperature (deposition source temperature) of approximately 50 to 600°C and a substrate temperature of approximately -50 to 300°C at a deposition rate of approximately 0.005 to 50 nm/sec. In this case, the respective layers of the hole injection transport layer, the luminescent layer, the electron injection transport layer and the like are formed continuously in vacuum so that the organic electroluminescent element having more excellent properties can be prepared. In the case that the respective layers of the hole injection transport layer, the luminescent layer, the electron injection transport layer, etc. are formed using a plurality of compounds, it is preferred to perform co-deposition by separately controlling temperatures for the respective boats charged with these compounds.

In the case that the respective layers are formed by the solution-coating process, the components which constitute the respective layers or the components which constitute the respective layers and binder resins, etc. are dissolved or dispersed in solvents, which are made the coating liquid. Examples of the binder resins, which can be used for the respective layers of the hole injection transport layer, the luminescent layer and the electron injection transport layer, include high molecular compounds such as poly-N-vinylcarbazole, polyallylate, polystyrene, polyester, polysiloxane, polymethyl acrylate, polymethyl methacrylate, polyether, polycarbonate, polyamide, polyimide, polyamideimide, poly(p-xylene), polyethylene, polyethylene ether, polypropylene ether, polyphenylene oxide, polyether sulfone, polyaniline and its derivatives, polythiophene and its derivatives, polyphenylenevinylene and its derivatives, polyfluorene and its derivatives, polythienylenevinylene and its derivatives, etc. The binder resins may be used solely or with more than one.

When the respective layers are formed by the solution-coating process, the components which constitute the respective layers or the components which constitute the respective layers and binder resins, etc. are dissolved or dispersed in suitable organic solvents (for example, hydrocarbon type solvents such as hexane, octane, decane, toluene, xylene, ethylbenzene, 1-methylnaphthalen, etc.; ketone type solvents such as acetone, methyl ethyl ketone, methyl isobutyl ketone, cyclohexanone, etc.; halogenated hydrocarbon type solvents such as dichloromethane, chloroform, tetrachloromethane, dichloroethane, trichloroethane, tetrachloroethane, chlorobenzene, dichlorobenzene, chlorotoluene, etc.; ester type solvents such as ethyl acetate, butyl acetate, amyl acetate, etc.; alcohol type solvents such as methanol, ethanol, propanol, butanol, pentanol, hexanol, cyclohexanol, methyl cellosolve, ethyl cellosolve, ethylene glycol, etc.; ether type solvents such as dibutyl ether, tetrahydrofuran, dioxane, anisole, etc.; polar solvents such as N,N-dimethylformamide, N,N-dimethylacetamide, 1-methyl-2-pyrrolidone, 1,3-dimethyl-2-imidazolidinone, dimethyl sulfoxide, etc.) and/or water. The resulting solution or dispersion is used as the coating liquid and by applying various coating techniques, thin films can be formed.

Techniques for dispersion are not particularly limited but using, e.g., ball mills, sand mills, a paint shaker, an attritor, a homogenizer, etc., the components, etc. can be ground into fine particles. Concentration of the coating liquid is not particularly limited but can be set out in a concentration range suitable for preparing a desired thickness, depending on coating methods performed. In general, the concentration of the solution is in the range of about 0.1-50% by weight, preferably about 1-30% by weight. When the binder resin is used, the amount of the binder resin is not particularly limited but in general, the amount is set in about 5-99.9% by weight, preferably about 10-99% by weight, and more preferably about 15-90% by weight, based on the total amount of the components which constitute the respective layers (i.e. when a single-layered type element is formed, the amount is set in the range based on the total amount of the components).

The layer thickness of the hole injection transport layer, the luminescent layer and the electron injection transport layer is not particularly limited but in general, it is preferred to set in about 5 nm to about 5 μm. For the purpose of preventing the element from contact with oxygen, moisture, etc. a protective layer (sealing layer) can be provided on the element thus prepared; alternatively, the element can be sealed into an inert substance such as paraffin, liquid paraffin, silicone oil, fluorocarbon oil, zeolite-containing fluorocarbon oil, etc. to protect the element.

Materials used for the protective layer are, for example, organic high molecular materials (e.g., fluorinated resin, epoxy resin, silicone resin, epoxy-silicone resin, polystyrene, polyester, polycarbonate, polyamide, polyimide, polyamideimide, poly(p-xylene), polyethylene, polyphenylene oxide), inorganic materials (e.g., diamond thin film, amorphous silica, electroinsulative glass, metal oxides, metal nitrides, metal carbides, metal sulfides), and furthermore, photocurable resin, etc. These materials used for the protective layer may be used solely or with more than one. The protective layer may be a single-layered structure or a multi- layered structure.

Also, a metal oxide film (e.g., an aluminum oxide film) or a metal fluoride film may be provided on the electrode as a protective layer. Furthermore, an interface layer (intermediate layer) composed of, e.g., an organophosphorous compound, polysilane, an aromatic amine derivative, a phthalocyanine derivative (e.g., copper phthalocyanine) or carbon can be provided on the anode. In addition, the electrodes, e.g., the anode can be surface-treated with, e.g., an acid, ammonia/hydrogen peroxide or plasma, and then provided for use.

The organic electroluminescent element of the present invention is generally used as an element of direct current-driven type but can also be used as an element of pulse-driven type or alternating current-driven type. The voltage applied is generally about 2-30 V. The organic electroluminescent element of the present invention can be used for panel type light sources, various luminescent devices, various display devices, various markings, various sensors, etc.

EXAMPLES

Hereinafter the present invention will be described in more detail, with reference to EXAMPLE but definitely is not limited thereto.

Preparation of Reference Compound 1

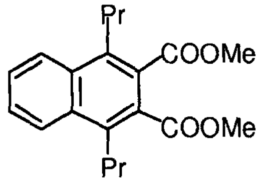

Dimethyl 1,4-dipropylnaphthalene-2,3-dicarboxylate

2,3-Dichloro-5,6-dicyanobenzoquinone (1.362 g, 6.0 mmol) was added to a solution of dimethyl 1,4-dipropyl-5,6,7,8-tetrahydronaphthalene-2,3-dicarboxylate (0.665 g, 2.0 mmol) in benzene (20 ml). Next, the mixture was refluxed for 24 hours. After filtration, the solvent in the mixture was removed in vacuum By column chromatography (ethyl acetate/hexane, 1/20) using silica gel, 0.464 g of the title compound was obtained as colorless crystals. The GC yield was 87% and the isolation yield was 71%.

Preparation of Reference Compound 2

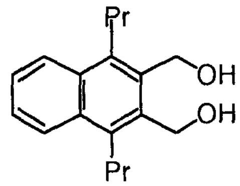

2,3-Bis(hydroxymethyl)-1,4-dipropylnaphthalene

Lithium aluminum hydride was added to a diethyl ether solution of dimethyl 1,4-dipropylnaphthalene-2,3-dicarboxylate obtained in Reference Compound 1 at 0°C. The mixture was then warmed to room temperature and stirred for an hour. Water was added thereto at room temperature to terminate the reaction. Thus, 0.219 g (0.898 mmol) of the title compound was obtained as a white solid. By recrystallization from ether/hexane, a small quantity of the title compound was obtained. The isolation yield was 90%.

Preparation of Reference Compound 3

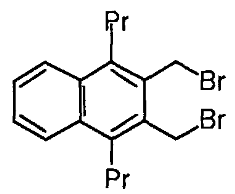

2,3-Bis(bromomethyl)-1,4-dipropylnaphthalene

After phosphorus tribromide (1 eq.) was added to a chloroform solution of 2,3-bis(hydroxymethyl)-1,4-dipropylnaphthalene obtained in Reference Compound 2 at room temperature, the mixture was stirred at room temperature for an hour. The mixture was then extracted with ether. After washing with brine, the extract was dried over anhydrous magnesium sulfate. The solvent was removed and the residue was subjected to column chromatography (ethyl acetate/hexane, 1/50) using silica gel to give 0.115 g (0.4 mmol) of the title compound as a white solid. The isolation yield was 72%.

Preparation of Reference Compound 4

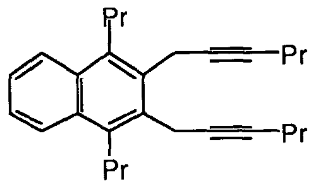

2,3-Bis(2-hexynyl)-1,4-dipropylnaphthalene

N,N'-Dimethylpropyleneurea (DMPU) and 1-pentynyl lithium were added to a THF solution of 2,3-bis(bromomethyl)-1,4-dipropylnaphthalene obtained in Reference Compound 3. The reaction mixture was stirred at room temperature for an hour. The reaction was terminated by adding 3N hydrochloric acid. Then, the mixture was extracted with ether. The extract was washed with sodium hydrogencarbonate aqueous solution and brine, followed by drying over anhydrous magnesium sulfate. The extract was concentrated under reduced pressure and by column chromatography (ethyl acetate/hexane, 1/50) using silica gel, 1.661 g (4.787 mmol) of the title compound was obtained as a white solid. The isolation yield was 93%.

Preparation of Reference Compound 5

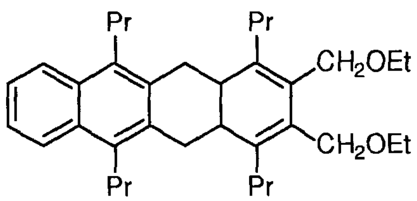

5,12-Dihydro-1,4,6,11-tetrapropylnaphthacene-2,3-diethoxymethyl

2,3-Bis(2-hexynyl)-1,4-dipropylnaphthalene obtained in Reference Compound 4 was added at -78°C to a THF solution of bis($\eta^5$-cyclopentadienyl)dibutylzirconium. The mixture was then warmed to room temperature and allowed to stand for an hour. Subsequently, $NiCl_2(PPh_3)_2$ and 1,4-diethoxy-2-butyne were added at room temperature to the reaction mixture as it was. The mixture was further stirred at room temperature for an hour. Then, 3N hydrochloric acid was added to terminate the reaction. Next, the reaction mixture was extracted with ether and the extract was washed with sodium hydrogencarbonate aqueous solution and brine, followed by drying over anhydrous magnesium sulfate. The extract was concentrated under reduced pressure and the residue was subjected to column chromatography (ethyl acetate/hexane, 1/10) using silica gel to give the title compound.

Preparation of Compound 1 of the present invention

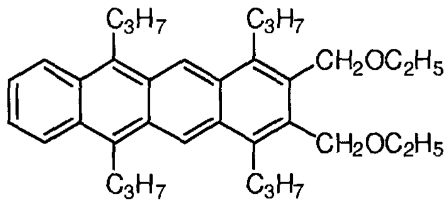

(Compound 1)

2,3-Bis(ethoxymethyl)-1,4,6,11-tetrapropylnaphthacene

5,12-Dihydro-1,4,6,11-tetrapropylnaphthacene-2,3-diethoxymethyl obtained as Reference Compound 5 was used. 2,3-Dichloro-5,6-dicyanobenzoquinone (0.050 g, 0.22 mmol) was added to a solution of 5,12-dihydro-1,4,6,11-tetrapropylnaphthacene-2,3-diethoxymethyl (0.2 mmol) in 1,4-dioxane (5 ml). Next, the mixture was refluxed for 3 hours. After filtration, the solvent in the mixture was removed in vacuum. After chloroform was added, the mixture was again filtered. Recrystallization from chloroform/methanol gave the title compound.

Preparation of Reference Compound 6

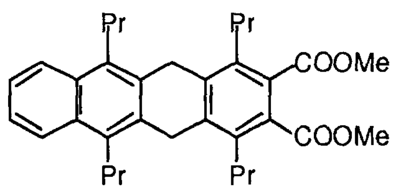

Dimethyl 5,12-dihydro-1,4,6,11-tetrapropylnaphthacene-2,3-dicarboxylate

2,3-Bis(2-hexynyl)-1,4-dipropylnaphthalene obtained in Reference Compound 4 was added at -78°C to a THF solution of bis($\eta^5$-cyclopentadienyl)dibutylzirconium. The mixture was then warmed to room temperature and allowed to stand for an hour. Next, CuCl and dimethylacetylene dicarboxylate were added at room temperature to the reaction mixture as it was, followed by stirring for further an hour at room temperature. Thereafter, 3N hydrochloric acid was added to terminate the reaction. Next, the reaction mixture was extracted with ether, and washed with sodium hydrogencarbonate aqueous solution and brine followed by drying over anhydrous magnesium sulfate. After concentrating under reduced pressure, the residue was subjected to column chromatography (ethyl acetate/hexane, 1/10) using silica gel to give 1.790 g (4.458 mmol) of the title compound as a light yellow solid. The isolation yield was 78%.

Preparation of Compound 2 of the present invention

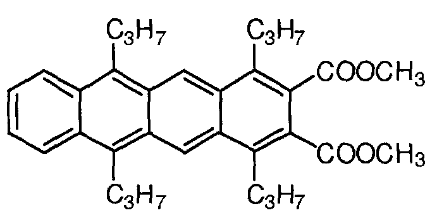

(Compound 2)

2,3-Bis(methoxycarbonyl)-1,4,6,11-tetrapropylnaphthacene

Dimethyl 5,12-dihydro-1,4,6,11-tetrapropylnaphthacene-2,3-dicarboxylate obtained in Reference Compound 6 was used. 2,3-Dichloro-5,6-dicyanobenzoquinone (0.050 g, 0.22 mmol) was added to a solution of dimethyl 5,12-dihydro-1,4,6,11-tetrapropylnaphthacene-2,3-dicarboxylate (0.103 g, 0.2 mmol) in 1,4-dioxane (5 ml). Thereafter, the mixture was refluxed for 3 hours. After filtration, the solvent in the mixture was removed in vacuum. Chloroform was added to the residue and the mixture was again filtered. Recrystallization from chloroform/methanol gave 0.076 g of the title compound as red needle-like crystals.

Preparation of Reference Compound 7

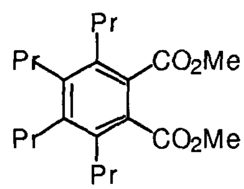

Dimethyl 3,4,5,6-tetrapropylphthalate

4-Octyne (5.9 ml, 40.0 mmol) was added at -78°C to 70 ml of a THF solution of bis($\eta^5$-cyclopentadienyl) dibutylzirconium, which was prepared from bis($\eta^5$-cyclopentadienyl)dichlorozirconium (7.016 g, 24.0 mmol) and n-butyl lithium (31.6 ml, 48.0 mmol, 1.52 M). After elevating to room temperature, the reaction mixture was stirred for an hour. DMAD (dimethylacetylene dicarboxylate) (7.4 ml, 60.0 mmol) and CuCl (3.96 g, 40.0 mmol) were added to the reaction mixture at room temperature. After stirring for an hour, 3N HCl was added for hydrolysis and the mixture was extracted with hexane. Then, the extract was washed with sodium hydrogencarbonate aqueous solution and brine in this order. After the extract was dried over anhydrous magnesium sulfate, column chromatography was performed using silica gel as the packing material to give the title compound (4.917 g) as light yellow oil. The GC yield was 82% and the isolation yield was 74%.

Preparation of Reference Compound 8

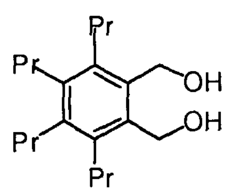

1,2-Bis(hydroxymethyl)-3,4,5,6-tetrapropylbenzene

Dimethyl 3,4,5,6-tetrapropylphthalate (5.22 g, 14.4 mmol) obtained in Reference Compound 7 was added at 0°C to a 50 ml THF solution of $LiAlH_4$ (1.20 g, 31.7 mmol). After stirring at room temperature for an hour, water was added for hydrolysis. The mixture was treated with 2N $H_2SO_4$ followed by extraction with diethyl ether. Subsequently, the extract was washed with brine and dried over anhydrous magnesium sulfate. Column chromatography was performed using silica gel as the packing material to give the title compound (3.67 g) as a white solid. The isolation yield was 91%.

Preparation of Reference Compound 9

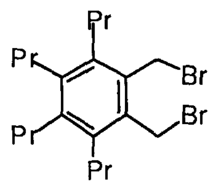

1,2-Bis(bromomethyl)-3,4,5,6-tetrapropylbenzene

Tribromophosphine (0.54 ml, 5.70 mmol) was dropwise added at room temperature to 20 ml of a chloroform solution of 1,2-bis(hydroxymethyl)-3,4,5,6-tetrapropylbenzene (1.75 g, 5.70 mmol) obtained in Reference Compound 8. After stirring for an hour, the mixture was treated with water followed by extracting with chloroform. Subsequently, the extract was washed with sodium hydrogencarbonate aqueous solution and brine, followed by drying over anhydrous magnesium sulfate. Column chromatography was performed using silica gel as the packing material to give the title compound (1.866 g) as a white solid. The GC yield was 100% and the isolation yield was 87%.

Preparation of Reference Compound 10

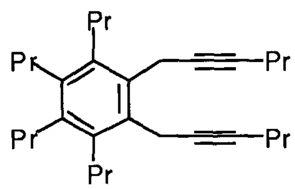

1,2-Bis(2-hexynyl)- 3,4,5,6- tetrapropylbenze ne

n-Butyl lithium (9.7 ml, 15.56 mmol, 1.6 M) was added to a 30 ml THF solution of 1-pentyne (1.67 ml, 17.12 mmol) at -78°C, and the mixture was stirred at room temperature for an hour. 1,2-Bis(bromomethyl)-3,4,5,6-tetrapropylbenzene (1.68 g, 3.89 mmol) obtained in Reference Compound 9 and DMPU (1.9 ml, 15.56 mmol) were added to the mixture at room temperature. After stirring for an hour, 3N HCl was added to terminate the reaction. The reaction mixture was extracted with hexane. The extract was then washed with sodium hydrogencarbonate aqueous solution and brine in this order, followed by drying over anhydrous magnesium sulfate. Column chromatography was performed using silica gel as the packing material to give the title compound (1.520 g) as a white solid. The GC yield was 100% and the isolation yield was 97%

Preparation of Reference Compound 11

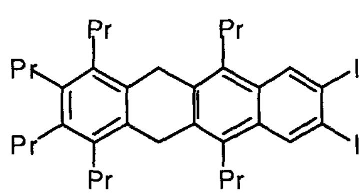

6,11-Dihydro-2,3-diiodo-5,7,8,9,10,12-hexapropylnaphthacene

n-Butyl lithium (3.0 ml, 4.8 mmol, 1.6 mol/l) was added to a solution of $Cp_2ZrCl_2$ (0.702 g, 2.4 mmol) in THF (20 ml) at -78°C. After the mixture was stirred for an hour, 1,2-bis(2-hexynyl)-3,4,5,6-tetrapropylbenzene (0.813 g, 2.0 mmol) obtained in Reference Compound 10 was added thereto. A cooling bath was withdrawn and the mixture was stirred for an hour. Tetraiodobenzene (1.16 g, 2.0 mmol), DMPU (0.73 ml, 6.0 mmol) and CuCl (0.416 g, 4.2 mmol) were added to the mixture. After the mixture was stirred at 50°C for an hour, 3N HCl was added thereto to terminate the reaction, followed by extraction with chloroform Next, the mixture was washed with sodium hydrogencarbonate aqueous solution and brine. After the pressure was reduced, column chromatography was performed using silica gel as the packing material to give the title compound (0.477 g) as a pink solid. The isolation yield was 33%.

Preparation of Compound 3 of the present invention

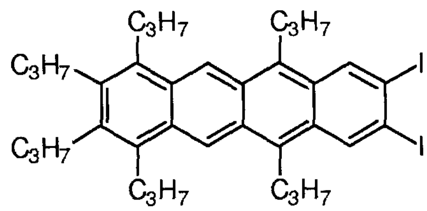

(Compound 3)

8,9-Diiodo-1,2,3,4,6,11-hexapropylnaphthacene

6,11-Dihydro-5,7,8,9,10,12-hexapropyl-2,3-diiodonaphthacene (0.238 g, 0.324 mmol) obtained in Reference Compound 11, 2,3-dichloro-5,6-dicyanobenzoquinone (0.081 g, 0.35 mmol) and 1,4-dioxane (2 ml) were charged in a reactor. The mixture was refluxed for 3 hours. After cooling, the precipitates were removed by filtration. The solvent in the mixture was removed in vacuum followed by recrystallization from chloroform/methanol. The title compound (0.081 g) of orange red was obtained. The isolation yield was 34%.

Preparation of Reference Compound 12

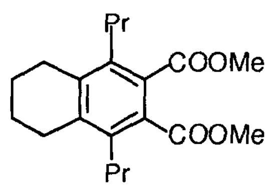

Dimethyl 1,4-dipropyl -5,6,7,8-tetrahydronaphthalene-2,3-dicarboxylate

4,10-Tetradodecadiyne (9.14 g, 48.03 mmol) was added at -78°C to a 200 ml THF solution of bis($\eta^5$-cyclopentadienyl)dibutylzirconium, which was prepared from bis($\eta^5$-cyclopentadienyl)dichlorozirconium (16.849 g, 57.64 mmol) and n-butyl lithium (75.8 ml, 115.3 mmol, 1.52 M). After elevating to room temperature, the reaction mixture was stirred for an hour. DMAD (17.4 ml, 144.01 mmol) and CuCl (9.51 g, 96.06 mmol) were added to the reaction mixture at room temperature. After stirring for an hour, 3N HCl was added for hydrolysis and the mixture was extracted with hexane. The extract was then washed with sodium hydrogencarbonate aqueous solution and brine in this order, followed by drying over anhydrous magnesium sulfate. Column chromatography was performed using silica gel as the packing material followed by recrystallization from methanol. The title compound (8.133 g) of colorless crystals was obtained. The GC yield was 58% and the isolation yield was 51%.

Preparation of Reference Compound 13

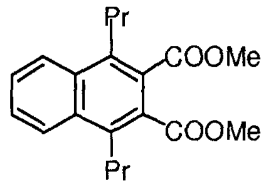

Dimethyl 1,4-dipropylnaphthalene -2,3-dicarboxylate

2,3-Dichloro-5,6-dicyanobenzoquinone (1.362 g, 6.0 mmol) was added to a benzene (20 ml) solution of dimethyl 1,4-dipropyl-5,6,7,8-tetrahydronaphthalene-2,3-dicarboxylate (0.665 g, 2.0 mmol) obtained in Reference Compound 12. The mixture was then refluxed for 24 hours. After filtration, the solvent in the mixture was removed in vacuum. Column chromatography was performed using silica gel as the packing material to give the title compound (0.464 g ) as colorless crystals. The GC yield was 87% and the isolation yield was 71%.

Preparation of Reference Compound 14

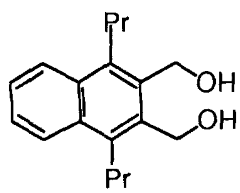

2,3-Bis(hydroxymethyl)-1,4-dipropylnaphthalene

Dimethyl 1,4-dipropylnaphthalene-2,3-dicarboxylate (0.295 g, 0.898 mmol) obtained in Reference Compound 13 was added to a 5 ml THF solution of $LiAlH_4$ (0.075 g, 1.98 mmol) at 0°C. After stirring at room temperature for an hour, water was added to effect hydrolysis. The mixture was treated with 2N $H_2SO_4$ followed by extraction with diethyl ether. The extract was washed with brine and dried over anhydrous magnesium sulfate. The extract was concentrated under reduced pressure. The title compound (0.219 g) was obtained as a white solid. The isolation yield was 90%.

Preparation of Reference Compound 15

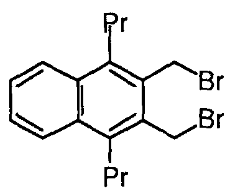

2,3-Bis(bromomethyl)-1,4-dipropylnaphthalene

Tribromophosphine (0.04 ml, 0.42 mmol) was dropwise added at room temperature to a 5 ml chloroform solution of 2,3-bis(hydroxymethyl)-1,4-dipropylnaphthalene (0.109 g, 0.40 mmol) obtained in Reference Compound 14. After stirring for an hour, the mixture was treated with water followed by extracting with chloroform. The extract was washed with sodium hydrogencarbonate aqueous solution and brine, followed by drying over anhydrous magnesium sulfate. Column chromatography was performed using silica gel as the packing material to give the title compound (0.115 g) as a white solid. The isolation yield was 72%.

Preparation of Reference Compound 16

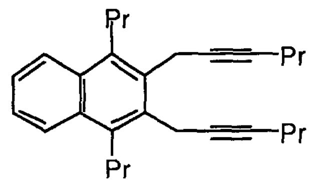

2,3-Bis (2-hexynyl)-1,4-dipropylnaphthalene

n-Butyl lithium (7.6 ml, 19.1 mmol, 2.52 M) was added to a 30 ml THF solution of 1-pentyne (2.05 ml, 21.06 mmol) at -78°C, and the mixture was stirred at room temperature for an hour. 2,3-Bis(bromomethyl)-1,4-dipropylnaphthalene (1.91 g, 4.79 mmol) obtained in Reference Compound 15 and DMPU (2.3 ml, 19.1 mmol) were added to the mixture at room temperature. After stirring for an hour, the mixture was treated with 3N HCl and extracted with hexane. The extract was then washed with sodium hydrogencarbonate aqueous solution and brine, followed by drying over anhydrous magnesium sulfate. Column chromatography was performed using silica gel as the packing material to give the title compound (1.66 g) as a white solid. The isolation yield was 93%.

Preparation of Reference Compound 17

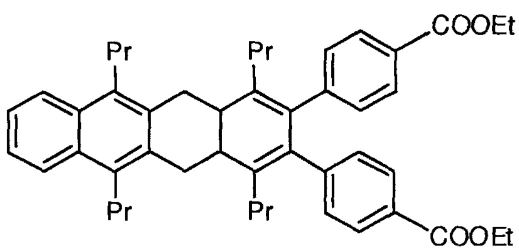

2,3-Bis(ethoxycarbonylphenyl)-5,12-dihydro-1,4,6,11-tetrapropylnaphthacene

2,3-Bis(2-hexynyl)-1,4-dipropylnaphthalene (0.373 g, 1.0 mmol) obtained in Reference Compound 16 was added at -78°C to a 20 ml THF solution of bis($\eta^5$-cyclopentadienyl)dibutylzirconium, which was prepared from bis($\eta^5$-cyclopentadienyl)dichlorozirconium (0.351 g, 1.2 mmol) and n-butyl lithium (1.5 ml, 2.4 mmol, 1.6 M). After elevating to room temperature, the reaction mixture was stirred for an hour. Bis(ethoxycarbonylphenyl)acetylene (1.5 mmol) and $NiBr_2(PPh_3)_2$ (0.892 g, 1.2 mmol) were added to the reaction mixture at room temperature. After stirring for 24 hours, hydrolysis was effected by 3N HCl followed by extraction with hexane. The extract was washed with sodium hydrogencarbonate aqueous solution and brine followed by drying over anhydrous magnesium sulfate. Column chromatography was performed using silica gel as the packing material to give the title compound.

Preparation of Compound 4 of the present invention

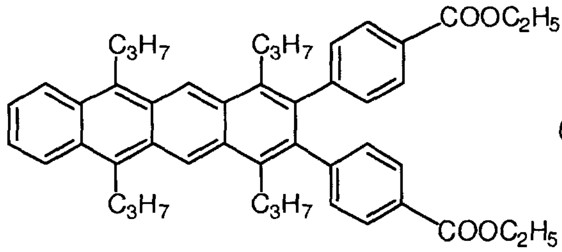

(Compound 4)

2,3-Bis(4-ethoxycarbonylphenyl)-1,4,6,11-tetrapropylnaphthacene

2,3-Bis(ethoxycarbonylphenyl)-5,12-dihydro-1,4,6,11-tetrapropylnaphthacene (1.04 mmol) obtained in Reference Compound 17, 2,3-dichloro-5,6-dicyanobenzoquinone (0.260 g, 1.14 mmol) and 1,4-dioxane (3 ml) were charged in a reactor. The mixture was refluxed for 24 hours. After cooling, the precipitates were removed by filtration. The solvent in the mixture was removed in vacuum followed by recrystallization from chloroform/methanol. The title compound was obtained.

Preparation of Reference Compound 18

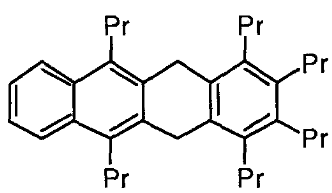

5,12-Dihydro -1,2,3,4,6,11-hexapropylnaphthacene

2,3-Bis(2-hexynyl)-1,4-dipropylnaphthalene (0.373 g, 1.0 mmol) obtained in Reference Compound 16 was added at -78°C to a 20 ml THF solution of bis($\eta^5$-cyclopentadienyl)dibutylzirconium, which was prepared from bis($\eta^5$-cyclopentadienyl)dichlorozirconium (0.351 g, 1.2 mmol) and n-butyl lithium (1.5 ml, 2.4 mmol, 1.6 M). After elevating to room temperature, the reaction mixture was stirred for an hour. 4-Octyne (0.22 ml, 1.5 mmol) and $NiBr_2(PPh_3)_2$ (0.892 g, 1.2 mmol) were added to the reaction mixture at room temperature. After stirring for 24 hours, hydrolysis was performed by 3N HCl followed by extraction with hexane. The extract was washed with sodium hydrogencarbonate aqueous solution and brine followed by drying over anhydrous magnesium sulfate. Column chromatography was performed using silica gel as the packing material to give the title compound (0.224 g) as somewhat orange powders by flouring with ethanol. The isolation yield was 46%.

Preparation of Compound 5 of the present invention

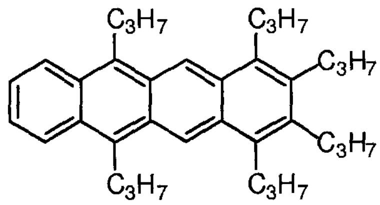

(Compound 5)

1,2,3,4,6,11-Hexapropylnaphthacene

5,12-Dihydro-1,2,3,4,6,11-hexapropylnaphthacene (0.503 g, 1.04 mmol) obtained in Reference Compound 18, 2,3-dichloro-5,6-dicyanobenzoquinone (0.260 g, 1.14 mmol) and 1,4-dioxane (3 ml) were charged in a reactor. The mixture was refluxed for 24 hours. After cooling, the precipitates were removed by filtration. The solvent in the mixture was removed in vacuum followed by recrystallization from chloroform/methanol. The title compound (0.112 g) of orange red was obtained. The NMR yield was 36% and the isolation yield was 22%.

Preparation of Reference Compound 19

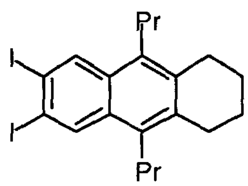

9,10-Dipropyl-2,3-diiodo-5,6,7,8-tetrahydroanthracene

n-Butyl lithium (0.75 ml, 1.2 mmol, 1.6 mol/l) was added at -78°C to a solution of bis($\eta^5$-cyclopentadienyl) dichlorozirconium (0.175 g, 0.6 mmol) in THF (25 ml). After stirring the solution for an hour, 4,10-tetradodecadiyne (0.095 ml, 0.5 mmol) was added to the solution. A cooling bath was withdrawn and the mixture was stirred for an hour. Tetraiodobenzene (0.582 g, 1.0 mmol), DMPU (0.18 ml, 1.5 mmol) and CuCl (0.104 g, 1.1 mmol) were added to the mixture. After stirring at 50°C for an hour, 3N hydrochloric acid was added to terminate the reaction. Next, the mixture was extracted with ether followed by washing with sodium hydrogencarbonate aqueous solution and brine. After concentrating under reduced pressure, the residue was subjected to column chromatography using silica gel as the packing material to give the title compound (0.148 g) as a colorless solid. The isolation yield was 57%.

Preparation of Reference Compound 20

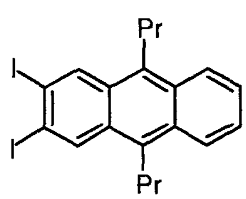

9,10-Dipropyl-2,3-diiodoanthracene

9,10-Dipropyl-2,3-diiodo-5,6,7,8-tetrahydroanthracene (0.259 g, 0.5 mmol) obtained in Reference Compound 19, 2,3-dichloro-5,6-dicyanobenzoquinone (0.341 g, 1.5 mmol) and 1,4-dioxane (3 ml) were charged in a reactor. The mixture was then refluxed for an hour. After cooling, the precipitates were removed by filtration. The solvent in the mixture was removed in vacuum. Column chromatography (hexane) was performed to give the title compound (0.109 g) as a light yellow solid. The isolation yield was 42%.

Preparation of Compound 6 of the present invention

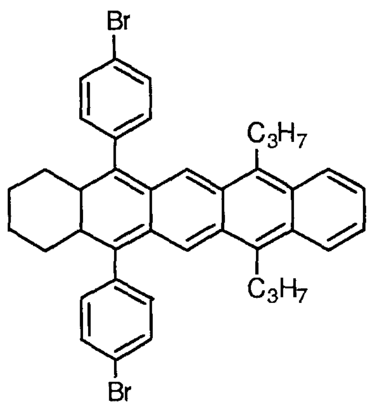

(Compound 6)

5,14-Bis(4-bromophenyl)-1,2,3,4-tetrahydro-7,12-dipropylpentacene

1,8-Bis(p-bromophenyl)-1,7-octadiyne (0.191 g, 0.459 mmol) was added at -78°C to a THF solution of bis($\eta^5$-cyclopentadienyl)dibutylzirconium, which was prepared from bis($\eta^5$-cyclopentadienyl)dichlorozirconium (0.161 g, 0.551 mmol) and n-butyl lithium (0.7 ml, 1.6 M, 1.1 mmol). The mixture was then allowed to stand at room temperature for an hour. CuCl (0.095 g, 0.964 mmol), DMPU (0.17 ml, 1.38 mmol) and 2,3-diiodo-9,10-dipropylanthracene (0.236 g, 0.459 mmol) obtained in Reference Compound 20 were added to the mixture. After heating at 50°C for an hour, the solvent in the mixture was removed in vacuum. Column chromatography (chloroform) was performed. Recrystallization from chloroform/methanol gave the title compound (0.177 g) as orange red. The isolation yield was 57%.

Comparative Compound 1

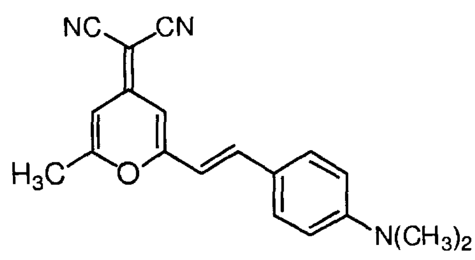

(Comparative Compound 1)

The title compound was purchased from Aldrich, Inc. and used as it was.

Comparative Compound 2

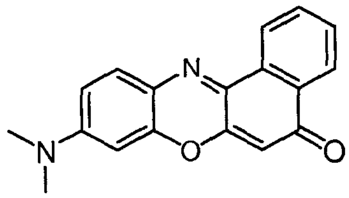

(Comparative Compound 2)

The title compound was purchased from Exciton, Inc. and used as it was.

Comparative Compound 3

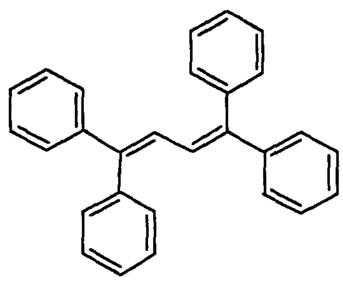

(Comparative Compound 3)

The title compound was purchased from Aldrich, Inc. and used as it was.

EXAMPLE 1

A glass substrate of 130 nm thick having an ITO transparent electrode was washed by ultrasonication using acetone, a substrate detergent, distilled water and isopropyl alcohol in this order. The substrate was further washed by UV/ozone and then fixed on a holder of deposition device. A deposition tank was depressurized to about $10^{-6}$ Torr. After TPD [N,N'-bis(3-methylphenyl)-N,N'-diphenylbenzidine] was deposited onto the ITO transparent electrode in a film thickness of about 30 nm, the compounds listed in TABLE 1, which were prepared as described above, and Alq. [tris(8-hydroxyquinolinato]aluminum] were deposited thereon in a weight ratio of 1 : 100 in a film thickness of about 40 nm. Then, Alq. alone was further deposited in a film thickness of about 20 nm. Next, a patterned mask (luminescent area was 5 mm x 5 mm) was applied onto the organic thin film, and magnesium and silver were co-deposited (weight ratio of 10 : 1) in a film thickness of about 150 nm to make the cathode. Thus, the organic EL element was prepared. A direct current voltage was applied to the organic EL element using Model 2400 source meter manufactured by KEITHLEY, Inc. to emit light. Its luminance and luminescent wavelength were measured using a luminance meter Model BM-9 of TOPCON Corp. and a multi-channel detector Model PMA-11 manufactured by Hamamatsu Photonics K.K., respectively. The results are shown in TABLE 1.

TABLE 1

| | Maximum Luminance (cd/$m^2$) | Driving Voltage (V) | Luminescent Wavelength (nm) |
|---|---|---|---|
| Compound 1 of the Present Invention | 2100 | 11 | 520 |
| Compound 2 of the Present Invention | 2000 | 12 | 525 |

TABLE 1 (continued)

| | Maximum Luminance (cd/m2) | Driving Voltage (V) | Luminescent Wavelength (nm) |
|---|---|---|---|
| Compound 4 of the Present Invention | 2600 | 13 | 520 |
| Compound 5 of the Present Invention | 2700 | 13 | 520 |
| Compound 6 of the Present Invention | 2800 | 14 | 525 |
| Comparative Compound 1 | 1400 | 14 | 600 |
| Comparative Compound 2 | 320 | 15 | 570 |

EXAMPLE 2

An ITO transparent electrode was washed and fixed on a substrate holder of deposition device in the same manner as used in EXAMPLE 1. A deposition tank was depressurized to about $10^{-6}$ Torr. On the ITO transparent electrode, TPD [N,N'-bis(3-methylphenyl)-N,N'-diphenylbenzidine], the compounds shown in TABLE 2 prepared as described above and Alq. [tris(8-hydroxyquinolinato]aluminum] were deposited in this order in a film thickness of about 30nm, 40 nm and 60 nm, respectively. Then, the cathode was deposited in the same manner as used in EXAMPLE 1, and evaluation was carried out. The results are shown in TABLE 2.

TABLE 2

| | Maximum Luminance (cd/m2) | Driving Voltage (V) |
|---|---|---|
| Compound 1 of the Present Invention | 2400 | 12 |
| Compound 2 of the Present Invention | 2200 | 11 |
| Compound 4 of the Present Invention | 2800 | 13 |
| Compound of the Present Invention 5 | 3000 | 13 |
| Compound of the Present Invention 6 | 3200 | 12 |
| Comparative Compound 1 | 1100 | 13 |

After 21 mg of PVK [poly(N-vinylcarbazole)], 9 mg of PBD [2-(4-biphenylyl)-5-(4-tert-butylphenyl)-1,3,4-oxadiazole] and 1 mg of the compound listed in TABLE 3 prepared as described above were dissolved in 3 ml of 1,2-dichloroethane, the solutions was spin-coated onto the ITO substrate which was washed in the same manner as used in EXAMPLE 1. The film thickness of the organic thin film was about 60 nm. The cathode (150 nm) was then deposited in the same manner as used in EXAMPLE 1 and evaluation was performed. The results are shown in TABLE 3.

TABLE 3

| | Maximum Luminance (cd/m2) | Driving Voltage (V) | Luminescent Wavelength (nm) |
|---|---|---|---|
| Compound 1 of the Present Invention | 1200 | 15 | 515 |
| Compound 2 of the Present Invention | 1100 | 16 | 525 |
| Compound 3 of the Present Invention | 1200 | 16 | 540 |
| Compound 4 of the Present Invention | 1300 | 17 | 520 |
| Compound 5 of the Present Invention | 1400 | 16 | 520 |

TABLE 3 (continued)

| | Maximum Luminance (cd/ $m^2$) | Driving Voltage (V) | Luminescent Wavelength (nm) |
|---|---|---|---|
| Compound 6 of the Present Invention | 1100 | 16 | 530 |
| Comparative Compound 1 | 700 | 16 | 600 |
| Comparative Compound 3 | 680 | 16 | 480 |

According to the present invention, there can be provided the materials for organic electroluminescent elements and the organic electroluminescent elements, which are excellent in stability, durability, luminance and luminance efficiency.

## Claims

**1.** A material for organic electroluminescent element selected from a polyacene derivative represented by general formula (I) below:

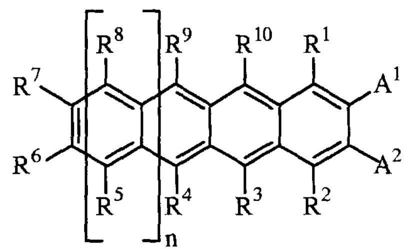

(I)

[wherein $R^1$, $R^2$, $R^3$, $R^4$, $R^5$, $R^6$, $R^7$, $R^8$, $R^9$ and $R^{10}$, which may be the same or different, independently represents hydrogen atom; a $C_1$-$C_{40}$ hydrocarbon group which may optionally be substituted; a $C_1$-$C_{40}$ alkoxy group which may optionally be substituted, a $C_6$-$C_{40}$ aryloxy group which may optionally be substituted; an amino group which may optionally be substituted, hydroxy group, or a silyl group which may optionally be substituted; provided that $R^6$ and $R^7$ may be cross-bridged with one another to form a $C_4$-$C_{40}$ saturated or unsaturated ring, and the saturated or unsaturated ring may be intervened by oxygen atom, sulfur atom or a group shown by formula: -N($R^{11}$)- (wherein $R^{11}$ is hydrogen atom or a hydrocarbon group) and may optionally be substituted;

$A^1$ and $A^2$, which may be the same or different, independently represents hydrogen atom; a halogen atom; a $C_1$-$C_{40}$ hydrocarbon group which may optionally be substituted; a $C_1$-$C_{40}$ alkoxy group which may optionally be substituted; a $C_6$-$C_{40}$ aryloxy group which may optionally be substituted; a $C_7$-$C_{40}$ alkylaryloxy group which may optionally be substituted; a $C_2$-$C_{40}$ alkoxycarbonyl group which may optionally be substituted; a $C_7$-$C_{40}$ aryloxycarbonyl group which may optionally be substituted; cyano group (-CN); carbamoyl group (-C(=O)$NH_2$); a halo formyl group (-C(=O)-X, wherein X represents a halogen atom); formyl group (-C(=O)-H); isocyano group; isocyanate group; thiocyanate group or thioisocyanate group; provided that $A^1$ and $A^2$ may be cross-bridged with each other to form a ring shown by formula: -C(=O)-B-C(=O)- (wherein B is oxygen atom or a group shown by formula -N($B^1$)- (wherein $B^1$ is hydrogen atom, a $C_1$-$C_{40}$ hydrocarbon group or a halogen atom));

n is an integer of at least 1;

provided that, the case wherein $R^1$, $R^2$, $R^3$, $R^4$, $R^5$, $R^6$, $R^7$, $R^8$, $R^9$, $R^{10}$, $A^1$ and $A^2$ are all hydrogen atoms is excluded,

the case wherein $R^7$ and $A^1$ are methoxy groups or $A^2$ and $R^6$ are methoxy groups is excluded,

when n is 1;

at least $R^1$, $R^2$, $R^4$ and $R^9$ are groups other than hydrogen atom, or at least $R^3$, $R^5$, $R^8$ and $R^{10}$ are groups other than hydrogen atom,

the case wherein $R^3$ and $R^{10}$ or $R^4$ and $R^9$ are an aryl group which may optionally be substituted is excluded; and

when n is 2, the formula (I) above is a pentacene derivative represented by formula (Ia) below:

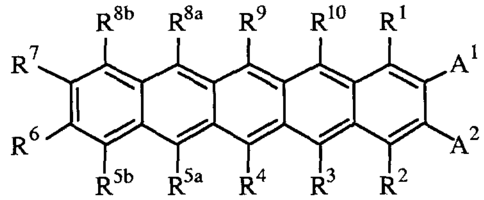

(Ia)

(wherein $A^1$ and $A^2$ have the same significance as described above,

$R^1$, $R^2$, $R^3$, $R^4$, $R^{5a}$, $R^{5b}$, $R^6$, $R^7$, $R^{8a}$, $R^{8b}$, $R^9$ and $R^{10}$, which may be the same or different, independently represents hydrogen atom; a $C_1$-$C_{40}$ hydrocarbon group which may optionally be substituted; a $C_1$-$C_{40}$ alkoxy group which may optionally be substituted; a $C_6$-$C_{40}$ aryloxy group which may optionally be substituted; an amino group which may optionally be substituted; a hydroxy group; or a silyl group which may optionally be substituted; provided that $R^6$ and $R^7$ maybe cross-bridged with each other to form a $C_4$-$C_{40}$ saturated or unsaturated ring, and the saturated or unsaturated ring may be intervened by oxygen atom, sulfur atom or a group represented by formula: $-N(R^{11})-$ (wherein $R^{11}$ is hydrogen atom or a hydrocarbon group) and may optionally be substituted) and;

the case in which at least one of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ is a diarylamine group is excluded).

**2.** The material for organic electroluminescent element as claimed in claim 1, which is represented by general formula (I) wherein n is 1 or 2;

when n is 1, the case wherein $R^1$, $R^2$, $R^3$, $R^4$, $R^5$, $R^6$, $R^7$, $R^8$, $R^9$, $R^{10}$, $A^1$ and $A^2$ are all methyl groups, the case wherein $R^3$, $R^4$, $R^9$ and $R^{10}$ are all aryl groups and $R^1$, $R^2$, $R^5$, $R^6$, $R^7$, $R^8$, $A^1$ and $A^2$ are all hydrogen atoms, the case wherein $R^1$, $R^2$, $R^4$ and $R^9$ are all alkoxy groups or aryloxy groups and $R^3$, $R^5$, $R^6$, $R^7$, $R^8$, $R^{10}$, $A^1$ and $A^2$ are all hydrogen atoms, and the case wherein $R^3$, $R^5$, $R^8$ and $R^{10}$ are all alkoxy groups or aryloxy groups and $R^1$, $R^2$, $R^4$, $R^6$, $R^7$, $R^9$, $A^1$ and $A^2$ are all hydrogen atoms, are excluded;

when n is 2, the polyacene derivative is a pentacene derivative represented by the formula (Ia) above and the following cases are excluded:

the case that in the formula (Ia); $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are all methyl groups; or $R^1$, $R^2$, $R^3$, $R^4$, $R^{5a}$, $R^{5b}$, $R^{8a}$, $R^{8b}$, $R^9$ and $R^{10}$ are all hydrogen atoms and at least one of $R^6$, $R^7$, $A^1$ and $A^2$ is an aryl group; or when at most 6 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom, any one of the groups other than hydrogen atom is methoxy group; the case of a pentacene derivative represented by formula (Ib) below:

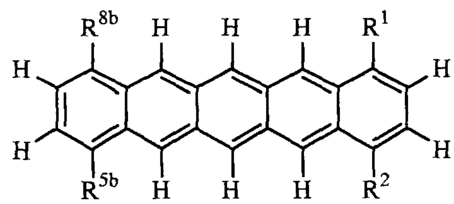

(Ib)

[wherein $R^1$, $R^2$, $R^{5b}$ and $R^{8b}$ are all alkoxy groups or aryloxy groups];

the case of a pentacene derivative represented by formula (Ic):

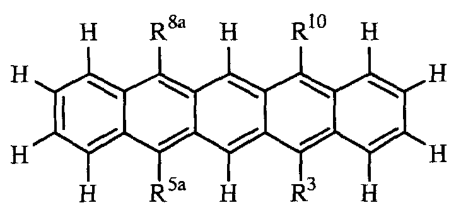

(Ic)

[wherein at least 2 of $R^3$, $R^{5a}$, $R^{8a}$ and $R^{10}$ are aryl groups or arylalkynyl groups, or at least one of $R^3$, $R^{5a}$, $R^{8a}$ and $R^{10}$ is an arylalkenyl group, or $R^3$, $R^{5a}$, $R^{8a}$ and $R^{10}$ are all alkoxy groups or aryloxy groups]; and,

the case of a pentacene derivative represented by formula (Id) below:

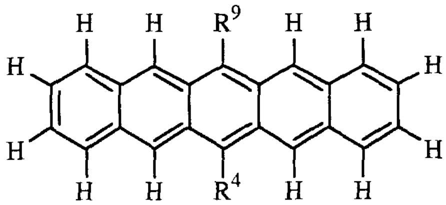

(Id)

[wherein $R^4$ and $R^9$ are hydrogen atom, a hydrocarbon group, an alkoxy group, an aryloxy group, or a halogen atom or hydroxy group].

**3.** The material for organic electroluminescent element as claimed in claim 1 or 2, wherein at least 5 of $R^1$, $R^2$, $R^3$, $R^4$, $R^5$, $R^6$, $R^7$, $R^8$, $R^9$, $R^{10}$, $A^1$ and $A^2$ are groups other than hydrogen atom.

**4.** The material for organic electroluminescent element as claimed in claim 1 or 2, wherein at least 6 of $R^1$, $R^2$, $R^3$, $R^4$, $R^5$, $R^6$, $R^7$, $R^8$, $R^9$, $R^{10}$, $A^1$ and $A^2$ are groups other than hydrogen atom.

**5.** The material for organic electroluminescent element as claimed in claim 1 or 2, wherein the polyacene derivative is a pentacene derivative represented by the formula (Ia) above; and,

at least 5 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom.

**6.** The material for organic electroluminescent element as claimed in claim 5, wherein at least 6 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom.

**7.** The material for organic electroluminescent element as claimed in claim 5, wherein at least 7 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom.

**8.** The material for organic electroluminescent element as claimed in claim 5, wherein at least 8 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom.

**9.** The material for organic electroluminescent element as claimed in claim 5, wherein at least 9 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom.

**10.** The material for organic electroluminescent element as claimed in claim 5, wherein at least 10 of $R^1$, $R^2$, $R^3$, $R^4$, $R^{5a}$, $R^{5b}$, $R^6$, $R^7$, $R^{8a}$, $R^{8b}$, $R^9$, $R^{10}$, $A^1$ and $A^2$ are groups other than hydrogen atom.

**11.** The material for organic electroluminescent element as claimed in any one of claims 1 to 4, wherein any combination of $R^1$ and $R^2$, $R^3$ and $R^{10}$, $R^4$ and $R^9$, $R^5$ and $R^8$, $R^6$ and $R^7$ as well as $A^1$ and $A^2$ is the same substituent.

**12.** The material for organic electroluminescent element as claimed in any one of claims 5 to 10, wherein any combination of $R^1$ and $R^2$, $R^3$ and $R^{10}$, $R^4$ and $R^9$, $R^{5a}$ and $R^{8a}$, $R^{5b}$ and $R^{8b}$, $R^6$ and $R^7$ as well as $A^1$ and $A^2$ is the same substituent.

**13.** The material for organic electroluminescent element as claimed in any one of claims 1 to 4, wherein any one of $R^1$, $R^2$, $R^3$, $R^4$, $R^5$, $R^6$, $R^7$, $R^8$, $R^9$ and $R^{10}$ is a $C_1$-$C_{40}$ hydrocarbon group which may optionally be substituted; a $C_1$-$C_{40}$ alkoxy group which may optionally be substituted, or a $C_6$-$C_{40}$ aryloxy group which may optionally be substituted.

**14.** The material for organic electroluminescent element as claimed in any one of claims 5 to 10, wherein any one of $R^1$, $R^2$, $R^3$, $R^4$, $R^{5a}$, $R^{5b}$, $R^6$, $R^7$, $R^{8a}$, $R^{8b}$, $R^9$ and $R^{10}$ is a $C_1$-$C_{40}$ hydrocarbon group which may optionally be substituted; a $C_1$-$C_{40}$ alkoxy group which may optionally be substituted, or a $C_6$-$C_{40}$ aryloxy group which may

optionally be substituted.

**15.** The material for organic electroluminescent element as claimed in claim 1, wherein $R^1$, $R^2$, $R^4$ and $R^9$, which may be the same or different, independently represents a $C_1$-$C_{40}$ alkyl group which may optionally be substituted or a $C_6$-$C_{18}$ aryl group which may optionally be substituted;
$A^1$ and $A^2$, which may be the same or different, independently represents a $C_2$-$C_{40}$ alkoxycarbonyl group which may optionally be substituted; and,
n is 1.

**16.** The material for organic electroluminescent element as claimed in claim 1, wherein $A^1$, $A^2$, $R^1$, $R^2$, $R^4$ and $R^9$, which may be the same or different, independently represents a $C_1$-$C_{40}$ alkyl group which may optionally be substituted or a $C_6$-$C_{18}$ aryl group which may optionally be substituted; and,
n is 1.

**17.** The material for organic electroluminescent element as claimed in claim 1, wherein $R^3$, $R^5$, $R^6$, $R^7$, $R^8$ and $R^{10}$, which may be the same or different, independently represents a $C_1$-$C_{40}$ alkyl group which may optionally be substituted or a $C_6$-$C_{18}$ aryl group which may optionally be substituted;
$A^1$ and $A^2$, which may be the same or different, independently represents a halogen atom; and,
n is 1.

**18.** The material for organic electroluminescent element as claimed in claim 1 or 2, wherein, when the polyacene derivative is a pentacene derivative represented by the formula (Ia) above, $A^1$ and $A^2$ represent a $C_2$-$C_{40}$ alkoxycarbonyl group which may optionally be substituted, and, $R^1$, $R^2$, $R^4$, $R^{5b}$, $R^6$, $R^7$, $R^{8b}$ and $R^9$ represent a $C_1$-$C_{40}$ alkyl group which may optionally be substituted or a $C_6$-$C_{18}$ aryl group which may optionally be substituted.

**19.** The material for organic electroluminescent element as claimed in claim 1 or 2, wherein, when the polyacene derivative is a pentacene derivative represented by the formula (Ia) above, $A^1$, $A^2$, $R^1$, $R^2$, $R^4$, $R^{5b}$, $R^6$, $R^7$, $R^{8b}$ and $R^9$ represent a $C_1$-$C_{40}$ alkyl group which may optionally be substituted or a $C_6$-$C_{18}$ aryl group which may optionally be substituted.

**20.** The material for organic electroluminescent element as claimed in claim 1 or 2, wherein, when the polyacene derivative is a pentacene derivative represented by the formula (Ia) above, $A^1$ and $A^2$ represent a halogen atom, and, $R^3$, $R^{5a}$, $R^{8a}$ and $R^{10}$ represent a $C_1$-$C_{40}$ alkyl group which may optionally be substituted or a $C_6$-$C_{18}$ aryl group which may optionally be substituted.

**21.** An organic electroluminescent element comprising a pair of electrodes and at least one layer containing at least one of the polyacene derivative as claimed in any one of Claims 1 to 20, sandwiched between the electrodes.

**22.** The organic electroluminescent element as claimed in claim 21, wherein the layer containing at least one of the polyacene derivative is a luminescent layer.

**23.** The organic electroluminescent element as claimed in claim 21, wherein the layer containing at least one of the polyacene derivative is a hole injection transport layer.

**24.** The organic electroluminescent element as claimed in claim 21, wherein the layer containing at least one of the polyacene derivative is an electron injection transport layer.

**25.** The organic electroluminescent element as claimed in any one of claims 21 to 24, which further contains at least one of a polycyclic aromatic compound, a luminescent organic metal-complex or a triarylamine derivative.

**26.** The organic electroluminescent element as claimed in claims 21 to 25, wherein 0.1 to 40% by weight of the polyacene derivative as claimed in any one of claims 1 to 20 is contained in at least one layer.

# INTERNATIONAL SEARCH REPORT

International application No.
PCT/JP03/03670

A. CLASSIFICATION OF SUBJECT MATTER
Int.Cl$^7$ C09K11/06, H05B33/14, H05B33/22

According to International Patent Classification (IPC) or to both national classification and IPC

B. FIELDS SEARCHED

Minimum documentation searched (classification system followed by classification symbols)
Int.Cl$^7$ C09K11/06, H05B33/14, H05B33/22

Documentation searched other than minimum documentation to the extent that such documents are included in the fields searched

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| E,X | JP 2003-109764 A (Canon Inc.), 11 April, 2003 (11.04.03), (Family: none) | 1,2,13,14, 21-26 |
| E,X | JP 2003-105332 A (Canon Inc.), 09 April, 2003 (09.04.03), (Family: none) | 1,2,13,14, 21-26 |
| P,X | WO 02/100977 A1 (SANYO Electric Co., Ltd.), 19 December, 2002 (19.12.02), & JP 2003-55652 A | 1,2,11-14, 21-26 |
| P,X | JP 2002-167578 A (Toyo Ink Manufacturing Co., Ltd.), 11 June, 2002 (11.06.02), (Family: none) | 1,2,11-14, 21-26 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 June, 2003 (24.06.03) | 08 July, 2003 (08.07.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/03670

C (Continuation). DOCUMENTS CONSIDERED TO BE RELEVANT

| Category* | Citation of document, with indication, where appropriate, of the relevant passages | Relevant to claim No. |
|---|---|---|
| P,X | JP 2002-124385 A (Idemitsu Kosan Co., Ltd.), 26 April, 2002 (26.04.02), (Family: none) | 1,2,11-14, 21-26 |
| P,X | JP 2002-97465 A (Toyo Ink Manufacturing Co., Ltd.), 02 April, 2002 (02.04.02), (Family: none) | 1-14,16,17, 19-26 |
| P,X | JP 2002-93581 A (Denso Corp.), 29 March, 2002 (29.03.02), (Family: none) | 1,2,5-10,12, 14,19,21-26 |
| X | JP 2001-297883 A (Mitsubishi Chemical Corp.), 26 October, 2001 (26.10.01), Claims; page 5, (2-9) compounds (Family: none) | 1,2,11,13, 21-26 |
| X | JP 10-36832 A (Mitsubishi Chemical Corp.), 10 February, 1998 (10.02.98), Claims; tables 1, 2 (Family: none) | 1-14,21-26 |
| X | JP 2001-131434 A (Chemipro Kasei Kaisha, Ltd.), 15 May, 2001 (15.05.01), Claims (Family: none) | 1,2,11,13, 21-26 |
| X | JP 2000-268964 A (TDK Corp.), 29 September, 2000 (29.09.00), Claims (Family: none) | 1-14,21-26 |
| X | EP 999256 A1 (TDK Corp.), 10 May, 2000 (10.05.00), & WO 99/57220 A1 & WO 99/57221 A1 & JP 2000-26334 A & JP 2000-26337 A & EP 1000999 A1 & US 6387547 B1 | 1-14,21-26 |
| X | JP 2000-21571 A (Asahi Glass Co., Ltd.), 21 January, 2000 (21.01.00), Claims; Par. No. [0069] (Family: none) | 1,2,11,13, 21-26 |
| X | US 6203933 B1 (TDK Corp.), 20 May, 2001 (20.05.01), & JP 8-311442 A | 1-14,21-26 |
| X | JP 6-330032 A (Mitsubishi Kasei Corp.), 29 November, 1994 (29.11.94), Claims; tables 1, 2 (Family: none) | 1-14,21-26 |

Form PCT/ISA/210 (continuation of second sheet) (July 1998)

## INTERNATIONAL SEARCH REPORT

International application No.
PCT/JP03/03670

C (Continuation). DOCUMENTS CONSIDERED TO BE RELEVANT

| Category* | Citation of document, with indication, where appropriate, of the relevant passages | Relevant to claim No. |
|---|---|---|
| X | US 4775820 A (Canon Kabushiki Kaisha), 04 October, 1988 (04.10.88), & JP 61-37862 A | 1,2,13, 21-26 |
| X | JP 4-335087 A (Mitsubishi Kasei Corp.), 24 November, 1992 (24.11.92), Claims; Par. Nos. [0028] to [0029] (Family: none) | 1-14,21-26 |
| X | US 5077142 A (Ricoh Co., Ltd.), 13 December, 1991 (13.12.91), (Family: none) | 1,2,11-14, 16,21-26 |
| A | WO 01-64611 A1 (JAPAN SCIENCE AND TECHNOLOGY CO.), 07 September, 2001 (07.09.01), (Family: none) | 1-26 |

Form PCT/ISA/210 (continuation of second sheet) (July 1998)